(19) Europäisches Patentamt
European Patent Office
Office européen des brevets

(11) EP 2 501 379 B1

(12) **EUROPEAN PATENT SPECIFICATION**

(45) Date of publication and mention of the grant of the patent:
**23.03.2016 Bulletin 2016/12**

(21) Application number: **10832068.0**

(22) Date of filing: **17.11.2010**

(51) Int Cl.:
***A61K 31/397*** *(2006.01)* ***A61P 35/00*** *(2006.01)*

(86) International application number:
**PCT/US2010/056931**

(87) International publication number:
**WO 2011/062930 (26.05.2011 Gazette 2011/21)**

(54) **COMBINATION**
KOMBINATION
COMBINAISON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **17.11.2009 US 261813 P**

(43) Date of publication of application:
**26.09.2012 Bulletin 2012/39**

(73) Proprietor: **Novartis AG**
**4056 Basel (CH)**

(72) Inventors:
- **GILMER, Tona**
**Research Triangle Park**
**NC 27709 (US)**
- **KUMAR, Rakesh**
**Collegeville**
**PA 19426 (US)**
- **LAQUERRE, Sylvie**
**King Of Prussia**
**PA 19406 (US)**

(74) Representative: **Rudge, Sewkian et al**
**Novartis Pharma AG**
**Patent Department**
**4002 Basel (CH)**

(56) References cited:
**WO-A2-2004/004644 US-A1- 2006 014 768**

- **HJELMELAND ET AL: "The Combination of Novel Low molecular weight inhibitors of RAF and target of rapamycin decreases glioma prliferation and invasion.", MOLECULAR CANCER THERAPEUTICS, vol. 6, 31 August 2007 (2007-08-31), - 31 August 2007 (2007-08-31), pages 2449-2457, XP002698230,**
- **LEGRIER ET AL: "Targeting protein translation in human non small cell cancer via combined MEK and mammalian target of rapamycin suppression.", CANCER RES, vol. 67, 1 December 2007 (2007-12-01), - 1 December 2007 (2007-12-01), pages 11300-11308, XP002698231,**
- **HERSEY ET AL: "Small molecules and targeted therapies in sistant metastatic ease", ANNALS OF ONCOLOGY, vol. 20, no. suppl. 6, 1 August 2009 (2009-08-01), - 1 August 2009 (2009-08-01), pages vi35-vi40, XP002698232,**
- **JIN ET AL.: 'Dual Inhibition of Mitogen-Activated Protein Kinase Kinase and Mammalian Target of Rapamycin in Differentiated and Anaplastic Thyroid Cancer' J CLIN ENDOCRINOL METAB vol. 94, no. 10, October 2009, pages 4107 - 4112, XP008156575**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

The present invention relates to a method of treating cancer in a mammal and to combinations useful in such treatment. In particular, the method relates to a novel combination comprising the MEK inhibitor N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide, or a pharmaceutically acceptable salt or solvate thereof, with an mTOR inhibitor, pharmaceutical compositions comprising the same, and methods of using such combinations in the treatment of cancer.

### BACKGROUND OF THE INVENTION

Effective treatment of hyperproliferative disorders including cancer is a continuing goal in the oncology field. Generally, cancer results from the deregulation of the normal processes that control cell division, differentiation and apoptotic cell death. Apoptosis (programmed cell death) plays essential roles in embryonic development and pathogenesis of various diseases, such as degenerative neuronal diseases, cardiovascular diseases and cancer. One of the most commonly studied pathways, which involves kinase regulation of apoptosis, is cellular signaling from growth factor receptors at the cell surface to the nucleus (Crews and Erikson, Cell, 74:215-17, 1993).

Mitogen-activated protein (MAP) Kinase/extracellular signal-regulated kinase (ERK) kinase (hereinafter referred to as MEK) is known to be involved in the regulation of cell proliferation as a kinase that mediates Raf-MEK-ERK signal transduction pathway, and the Raf family (B-Raf, C-Raf etc.) activates the MEK family (MEK-1, MEK-2 etc.) and the MEK family activates the ERK family (ERK-1 and ERK-2).

Activation of Raf-MEK-ERK signal transduction pathway in cancer, particularly colorectal cancer, pancreatic cancer, lung cancer, breast cancer and the like, has been frequently observed.

In addition, since the signals produced by signal molecules such as growth factor, cytokine and the like converge to the activation of MEK-ERK, inhibitors of these functions are considered to more effectively suppress Raf-MEK-ERK signal transduction than suppression of the function of upstream kinases such as RTK, Ras, and Raf.

Moreover, it is also known that a compound having MEK inhibitory activity effectively induces inhibition of ERK1/2 activity and suppression of cell proliferation (The Journal of Biological Chemistry, vol. 276, No. 4, pp. 2686-2692, 2001), and the compound is expected to show effects on diseases caused by undesirable cell proliferation, such as tumor genesis and/or cancer.

The mammalian target of rapamycin (mTOR) also known as FK506 binding protein 12 rapamycin associated protein q (FRAP1) is a protein which in humans is encoded by the FRAP1 gene. mTOR is a serine/threonine protein kinase that regulates cell growth, cell proliferation, cell motility, cell survival, protein synthesis, and transcription.

Current research indicates that mTOR integrates the input from multiple upstream pathways including insulin, growth factors (such as IGF-1 and IGF-2), and mitogens. mTOR also functions as a sensor of cellular nutrient and energy levels and redox status. The disregulation of the mTOR pathway is implicated as a contributing factor to various human disease processes, especially various types of cancer. Rapamycin is a bacterial natural product that can inhibit mTOR through association with its intracellular receptor FKBP12. The FKBP12-rapamycin complex binds directly to the FKBP12-Rapamycin Binding (FRB) domain of the mTOR.

mTOR has been shown to function as the catalytic subunit of two distinct molecular complexes in cells, mTORC1 and mTORC2. mTOR inhibitors are already used in the treatment of transplant rejection. They are also beginning to be used for the treatment of cancer. mTOR inhibitors may also be useful for treating several age-associated diseases.

It would be useful to provide a novel therapy which provides more effective and/or enhanced treatment of an individual suffering the effects of cancer.

### SUMMARY OF THE INVENTION

The present inventors have identified combinations of chemotherapeutic agents that provide increased activity over monotherapy. In particular drug combinations that includes the MEK inhibitor: N-{3-[3-cyclopropyl-5-(2-fluoro-4-iodo-phenylamino)6,8-dimethyl;-2,4,7-trioxo-3,4,6,7-tetrahydro-2H-pyrido[4,3-d]pyrimidin-1-yl]phenyl}acetamide, or a pharmaceutically acceptable salt or solvate thereof, a compound of structure (I)

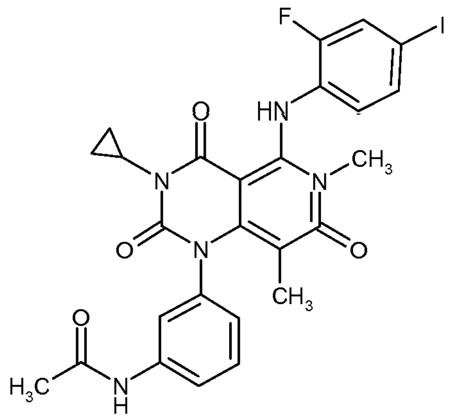

(I)

in combination with an mTOR inhibitor.

In a first aspect of the present invention, there is provided a combination comprising:

(i)N-{3-[3-cyclopropyl-5-(2-fluoro-4-iodo-phenylamino)6,8-dimethyl;-2,4,7-trioxo-3,4,6,7-tetrahydro-2H-pyrido[4,3-d]pyrimidin-1-yl]phenyl}acetamide (a compound of structure (I)):

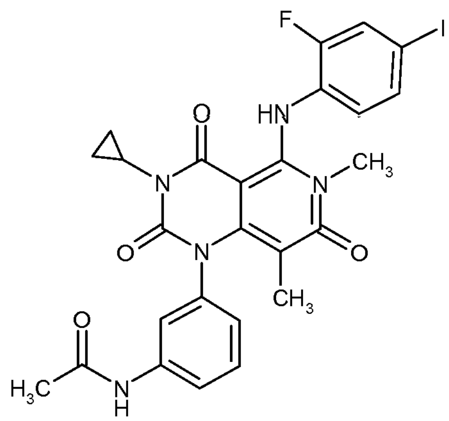

(I)

or a pharmaceutically acceptable salt or solvate, suitably the dimethyl sulfoxide solvate, thereof (hereinafter the dimethyl sulfoxide solvate of the compound of structure (I) is also referred to as Compound A, and the free or un-solvated form of the compound of structure (I) is also referred to as Compound C); everolimus

In a second aspect of the present invention, there is provided a combination, comprising:

(i) a compound of structure (I):

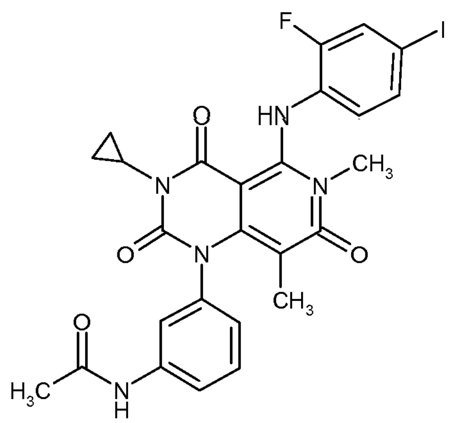

(I)

or a pharmaceutically acceptable salt or solvate, suitably the dimethyl sulfoxide solvate, thereof; and everolomus for use in therapy.

In a third aspect of the present invention, there is provided a combination, comprising:

(i) a compound of structure (I):

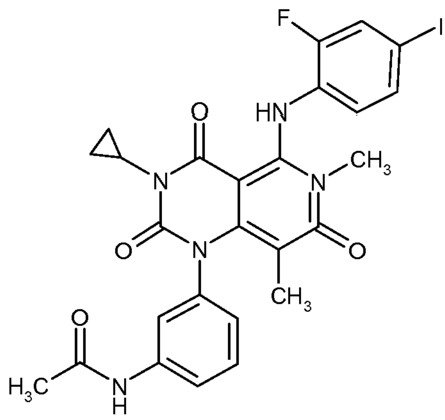

(I)

or a pharmaceutically acceptable salt or solvate, suitably the dimethyl sulfoxide solvate, thereof; and everolimus for use in the treatment of cancer.

In a fourth aspect of the present invention, there is provided a pharmaceutical composition, comprising:

(i) a compound of structure (I):

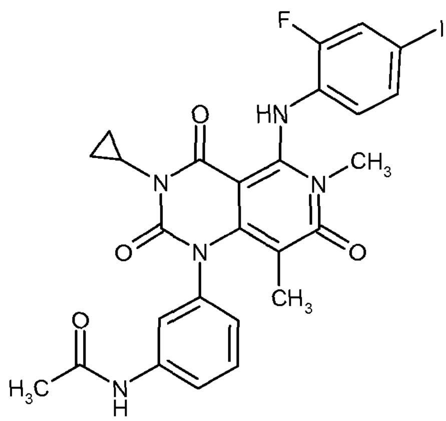

(I)

or a pharmaceutically acceptable salt or solvate, suitably the dimethyl sulfoxide solvate, thereof; and everolimus together with a pharmaceutically acceptable diluent or carrier.

In a fifth aspect there is provided the use of a combination comprising

i) a compound of formula (I)

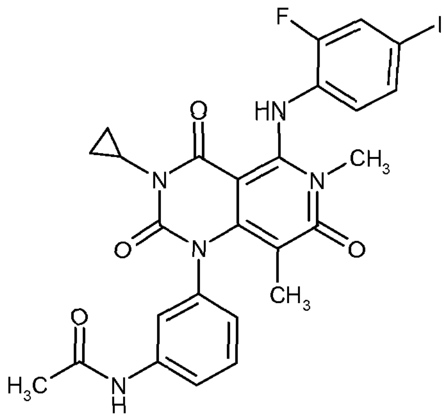

(I)

or a pharmaceutically acceptable salt or solvate, suitably the dimethyl sulfoxide solvate, thereof; and everolimus in the manufacture of a medicament for the treatment of cancer.

In a sixth aspect there is provided a method of treatment of cancer in a mammal comprising administering to said mammal:

(i) a therapeutically effective amount of a compound of formula (I)

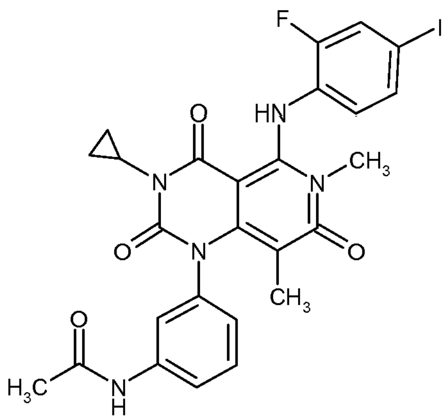

(I)

or a pharmaceutically acceptable salt or solvate, suitably the dimethyl sulfoxide solvate, thereof; and everolimus.

In a further aspect of this invention is provided a method of treating cancer in a mammal in need thereof which comprises administering a therapeutically effective amount of a combination of the invention wherein the compounds of the combination are administered within a specific period and for a duration of time.

In a further aspect of this invention is provided a method of treating cancer in a mammal in need thereof which comprises administering a therapeutically effective amount of a combination of the invention wherein the compounds of the combination are administered sequentially.

## BRIEF DESCRIPTION OF THE DRAWINGS

Figure - 1 Figure 1 depicts the cell growth inhibition-dose response curves for A427, A549, Calu6 and H2122 cell lines.

Figure - 2 Figure 2 depicts the caspase 3/7 activity curves for A427, A549, Calu6 and H2122 cell lines.

Figure - 3 Figure 3 depicts the growth IC50 (gIC50) of MEK and mTOR inhibitors alone and in combination against cancer cell lines.

Figure - 4 Figure 4 depicts the logarithmic value of combination index of MEK and mTOR inhibitors on cancer cell lines.

Figure - 5 Figure 5 depicts the effect of MEK inhibitor, mTOR inhibitor and their combination on A549 lung cancer cell line growth.

Figure - 6 Figure 6 depicts the cell response to MEK inhibitor, mTOR inhibitor and their combination on cancer cell lines.

## DETAILED DESCRIPTION OF THE INVENTION

The present invention relates to combinations that exhibit antiproliferative activity. Suitably, the method relates to methods of treating cancer by the co-administration of:

an mTOR inhibitor; and
N-{3-[3-cyclopropyl-5-(2-fluoro-4-iodo-phenylamino)-6,8-dimethy-2,4,7-trioxo-3,4,6,7-tetrahydro-2H-pyrido[4,3-d]pyrimidin-1-yl]phenyl}acetamide, or a pharmaceutically acceptable salt or solvate, suitably the dimethyl sulfoxide solvate, thereof,
which compound is represented by Structure I:

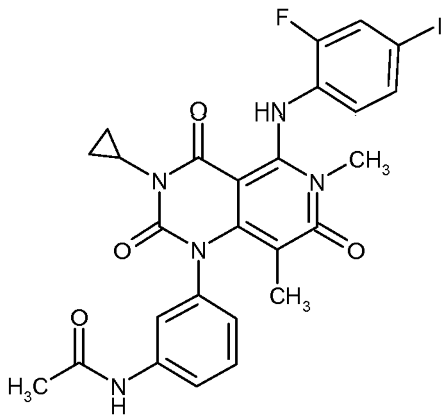

(I).

Compound C is disclosed and claimed, along with pharmaceutically acceptable salts and solvates thereof, as being useful as an inhibitor of MEK activity, particularly in treatment of cancer, in International Application No. PCT/JP2005/011082, having an International filing date of June 10, 2005; International Publication Number WO 2005/121142 and an International Publication date of December 22, 2005, B is the compound of Example 4-1. Compound C can be prepared as described in International Application No. PCT/JP2005/011082. Compound C can be prepared as described in United States Patent Publication No. US 2006/0014768, Published January 19, 2006, the entire disclosure of which is hereby incorporated by reference.

Suitably, Compound C is in the form of a dimethyl sulfoxide solvate, which is Compound A. Suitably, Compound C is in the form of a sodium salt. Suitably, Compound C is in the form of a hydrate or solvate selected from: acetic acid, ethanol, nitromethane, chlorobenzene, 1-pentanci, isopropyl alcohol, ethylene glycol and 3-methyl-1-butanol. These solvates and salt forms can be prepared by one of skill in the art from the description in International Application No. PCT/JP2005/011082 or United States Patent Publication No. US 2006/0014768.

For use herein, the term mTOR inhibitor, mTOR and derivatives is everolimus.

The administration of a therapeutically effective amount of the combinations of the invention are advantageous over the individual component compounds in that the combinations will provide one or more of the following improved properties when compared to the individual administration of a therapeutically effective amount of a component compound: i) a greater anticancer effect than the most active single agent, ii) synergistic or highly synergistic anticancer activity, iii) a dosing protocol that provides enhanced anticancer activity with reduced side effect profile, iv) a reduction in the toxic effect profile, v) an increase in the therapeutic window, vi) an increase in the bioavailability of one or both of the component compounds, or vii) an increase in apoptosis over the individual component compounds.

The compounds of the invention may contain one or more chiral atoms, or may otherwise be capable of existing as two enantiomers. Accordingly, the compounds of this invention include mixtures of enantiomers as well as purified enantiomers or enantiomerically enriched mixtures. Also, it is understood that all tautomers and mixtures of tautomers are included within the scope of Compound A, and pharmaceutically acceptable solvates and/or salts thereof, and an mTOR inhibiting compound, and pharmaceutically acceptable salts thereof.

The compounds of the invention may form a solvate which is understood to be a complex of variable stoichiometry formed by a solute (in this invention, Compound A or a salt or solvate thereof and/or an mTOR inhibitor or a salt thereof) and a solvent. Such solvents for the purpose of the invention may not interfere with the biological activity of the solute. Examples of suitable solvents include, but are not limited to, water, methanol, dimethyl sulfoxide, ethanol and acetic acid. Suitably the solvent used is a pharmaceutically acceptable solvent. Suitably the solvent used is not water.

The pharmaceutically acceptable salts of the compounds of the invention are readily prepared by those of skill in the art.

Also, contemplated herein is a method of treating cancer using a combination of the invention where an mTOR compound, or a pharmaceutically acceptable salt thereof, and/or Compound C or a pharmaceutically acceptable salt or solvate thereof are administered as pro-drugs. Pharmaceutically acceptable pro-drugs of the compounds of the invention are readily prepared by those of skill in the art.

When referring to a dosing protocol, the term "day", "per day" and the like, refer to a time within one calendar day which begins at midnight and ends at the following midnight.

By the term "treating" and derivatives thereof as used herein, is meant therapeutic therapy. In reference to a particular condition, treating means: (1) to ameliorate or prevent the condition of one or more of the biological manifestations of the condition, (2) to interfere with (a) one or more points in the biological cascade that leads to or is responsible for the condition or (b) one or more of the biological manifestations of the condition, (3) to alleviate one or more of the symptoms, effects or side effects associated with the condition or treatment thereof, or (4) to slow the progression of the condition or one or more of the biological manifestations of the condition. Prophylactic therapy is also contemplated thereby. The skilled artisan will appreciate that "prevention" is not an absolute term. In medicine, "prevention" is understood to refer to the prophylactic administration of a drug to substantially diminish the likelihood or severity of a condition or biological manifestation thereof, or to delay the onset of such condition or biological manifestation thereof. Prophylactic therapy is appropriate, for example, when a subject is considered at high risk for developing cancer, such as when a subject has a strong family history of cancer or when a subject has been exposed to a carcinogen.

As used herein, the term "effective amount" means that amount of a drug or pharmaceutical agent that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

By the term "combination" and derivatives thereof, as used herein is meant either, simultaneous administration or any manner of separate sequential administration of a therapeutically effective amount of an mTOR inhibiting compound and Compound C or a pharmaceutically acceptable salt or solvate thereof. Preferably, if the administration is not simultaneous, the compounds are administered in a close time proximity to each other. Furthermore, it does not matter if the compounds are administered in the same dosage form, e.g. one compound may be administered topically and the other compound may be administered orally. Suitably, both compounds are administered orally.

By the term "combination kit" as used herein is meant the pharmaceutical composition or compositions that are used to administer an mTOR inhibiting compound, and Compound C, or a pharmaceutically acceptable salt or solvate thereof, according to the invention. When both compounds are administered simultaneously, the combination kit can contain an mTOR inhibiting compound and Compound C, or a pharmaceutically acceptable salt or solvate thereof, in a single pharmaceutical composition, such as a tablet, or in separate pharmaceutical compositions. When the compounds are not administered simultaneously, the combination kit will contain an mTOR inhibiting compound and Compound C, or a pharmaceutically acceptable salt or solvate thereof, in separate pharmaceutical compositions. The combination kit can comprise an mTOR inhibiting compound and Compound C, or a pharmaceutically acceptable salt or solvate thereof, in separate pharmaceutical compositions in a single package or in separate pharmaceutical compositions in separate packages.

In one aspect there is provided a combination kit comprising the components:

an mTOR inhibiting compound in association with a pharmaceutically acceptable carrier; and
Compound C, or a pharmaceutically acceptable salt or solvate thereof, in association with a pharmaceutically acceptable carrier.

In one embodiment of the invention the combination kit comprises the following components:

an mTOR inhibiting compound in association with a pharmaceutically acceptable carrier; and

Compound C, or a pharmaceutically acceptable salt or solvate thereof, in association with a pharmaceutically acceptable carrier,

wherein the components are provided in a form which is suitable for sequential, separate and/or simultaneous administration.

In one embodiment the combination kit comprises:

a first container comprising an mTOR inhibiting compound in association with a pharmaceutically acceptable carrier; and

a second container comprising Compound C, or a pharmaceutically acceptable salt or solvate thereof, in association with a pharmaceutically acceptable carrier, and a container means for containing said first and second containers.

The "combination kit" can also be provided by instruction, such as dosage and administration instructions. Such dosage and administration instructions can be of the kind that is provided to a doctor, for example by a drug product label, or they can be of the kind that is provided by a doctor, such as instructions to a patient.

As used herein the term "neoplasm" refers to an abnormal growth of cells or tissue and is understood to include benign, i.e., non-cancerous growths, and malignant, i.e., cancerous growths. The term "neoplastic" means of or related to a neoplasm.

As used herein the term "agent" is understood to mean a substance that produces a desired effect in a tissue, system, animal, mammal, human, or other subject. Accordingly, the term "anti-neoplastic agent" is understood to mean a substance producing an anti-neoplastic effect in a tissue, system, animal, mammal, human, or other subject. It is also to be understood that an "agent" may be a single compound or a combination or composition of two or more compounds.

The compounds of the presently invented combinations may have the ability to crystallize in more than one form, a characteristic, which is known polymorphism, and it is understood that such polymorphic forms ("polymorphs") are within the scope of the compounds of the presently invented combinations. Polymorphism generally can occur as a response to changes in temperature or pressure or both and can also result from variations in the crystallization process. Polymorphs can be distinguished by various physical characteristics known in the art such as x-ray diffraction patterns, solubility, and melting point.

Unless otherwise defined, in all dosing protocols described herein, the regimen of compounds administered does not have to commence with the start of treatment and terminate with the end of treatment, it is only required that the number of consecutive days in which both compounds are administered and the optional number of consecutive days in which only one of the component compounds is administered, or the indicated dosing protocol - including the amount of compound administered, occur at some point during the course of treatment.

As used herein the term "Compound $C^2$" means ---Compound C, or a pharmaceutically acceptable salt or solvate thereof---.

The term "loading dose" as used herein will be understood to mean a single dose or short duration regimen of an mTOR inhibiting compound or Compound $C^2$ having a dosage higher than the maintenance dose administered to the subject to rapidly increase the blood concentration level of the drug. Suitably, a short duration regimen for use herein will be from: 1 to 14 days; suitably from 1 to 7 days; suitably from 1 to 3 days; suitably for three days; suitably for two days; suitably for one day. In some embodiments, the "loading dose" can increase the blood concentration of the drug to a therapeutically effective level. In some embodiments, the "loading dose" can increase the blood concentration of the drug to a therapeutically effective level in conjunction with a maintenance dose of the drug. The "loading dose" can be administered once per day, or more than once per day (e.g., up to 4 times per day). Suitably the "loading dose" will be administered once a day. Suitably, the loading dose will be an amount from 2 to 100 times the maintenance dose; suitably from 2 to 10 times; suitably from 2 to 5 times; suitably 2 times; suitably 3 times; suitably 4 times; suitably 5 times. Suitably, the loading dose will be administered for from 1 to 7 days; suitably from 1 to 5 days; suitably from 1 to 3 days; suitably for 1 day; suitably for 2 days; suitably for 3 days, followed by a maintenance dosing protocol.

The term "maintenance dose" as used herein will be understood to mean a dose that is serially administered (for example., at least twice), and which is intended to either slowly raise blood concentration levels of the compound to a therapeutically effective level, or to maintain such a therapeutically effective level. The maintenance dose is generally administered once per day and the daily dose of the maintenance dose is lower than the total daily dose of the loading dose.

Suitably the combinations of this invention are administered within a "specified period."

By the term "specified period" and derivatives thereof, as used herein is meant the interval of time between the administration of one of an mTOR inhibiting compound and Compound $C^2$ and the other of an mTOR inhibiting and Compound $C^2$. Unless otherwise defined, the specified period can include simultaneous administration. When both compounds of the invention are administered once a day the specified period refers to timing of the administration of an mTOR inhibiting and Compound $C^2$ during a single day. When one or both compounds of the invention are administered more than once a day, the specified period is calculated based on the first administration of each compound on a specific day. All administrations of a compound of the invention that are subsequent to the first during a specific day are not considered when calculating the specific period.

Suitably, if the compounds are not administered simultaneously, they will both be administered within 24 hours

of each other - in this case, the specified period will be 24 hours; suitably they will both be administered within 12 hours of each other - in this case, the specified period will be 12 hours; suitably they will both be administered within about 11 hours of each other - in this case, the specified period will be 11 hours; suitably they will both be administered within 10 hours of each other - in this case, the specified period will be 10 hours; suitably they will both be administered within 9 hours of each other - in this case, the specified period will be 9 hours; suitably they will both be administered within 8 hours of each other - in this case, the specified period will be 8 hours; suitably they will both be administered within 7 hours of each other - in this case, the specified period will be 7 hours; suitably they will both be administered within 6 hours of each other - in this case, the specified period will be 6 hours; suitably they will both be administered within 5 hours of each other - in this case, the specified period will be 5 hours; suitably they will both be administered within 4 hours of each other - in this case, the specified period will be 4 hours; suitably they will both be administered within 3 hours of each other - in this case, the specified period will be 3 hours; suitably they will be administered within 2 hours of each other - in this case, the specified period will be 2 hours; suitably they will both be administered within 1 hour of each other - in this case, the specified period will be about 1 hour. As used herein, the administration of an mTOR inhibiting compound and Compound $C^2$ in less than about 45 minutes apart is considered simultaneous administration.

Suitably, when the combination of the invention is administered for a "specified period", the compounds will be co-administered for a "duration of time".

By the term "duration of time" and derivatives thereof, as used herein is meant that both compounds of the invention are administered within a "specified period" for an indicated number of consecutive days, optionally followed by a number of consecutive days where only one of the component compounds is administered.

Regarding "specified period" administration:

Suitably, during the course of treatment, both compounds will be administered within a specified period for at least 1 day, followed by the administration of the mTOR inhibiting compound alone for at least 1 day - in this case, the duration of time will be at least 2 days; suitably, during the course of treatment, both compounds will be administered within a specified period for at least 1 day, followed by administration of the mTOR inhibiting compound alone for at least 2 days - in this case, the duration of time will be at least 3 days; suitably, during the course of treatment, both compounds will be administered within a specified period for at least 1 day, followed by administration of the mTOR inhibiting compound alone for at least 3 days - in this case, the duration of time will be at least 4 days; suitably, during the course of treatment, both compounds will be administered within a specified period for at least 1 day, followed by administration of the mTOR inhibiting compound alone for at least 4 days - in this case, the duration of time will be at least 5 days; suitably, during the course of treatment, both compounds will be administered within a specified period for at least 1 day, followed by administration of the mTOR inhibiting compound alone for at least 5 days - in this case, the duration of time will be at least 6 days; suitably, during the course of treatment, both compounds will be administered within a specified period for at least 1 day, followed by administration of the mTOR inhibiting compound alone for at least 6 days - in this case, the duration of time will be at least 7 days; suitably, during the course of treatment, both compounds will be administered within a specified period for at least 1 day, followed by administration of the mTOR inhibiting compound alone for at least 7 days - in this case, the duration of time will be at least 8 days; suitably, during the course of treatment, both compounds will be administered within a specified period for at least 2 consecutive days, followed by administration of the mTOR inhibiting compound alone for at least 1 day - in this case, the duration of time will be at least 3 days; suitably, during the course of treatment, both compounds will be administered within a specified period for at least 2 consecutive days, followed by administration of the mTOR inhibiting compound alone for at least 2 consecutive days - in this case, the duration of time will be at least 4 days; suitably, during the course of treatment, both compounds will be administered within a specified period for at least 2 consecutive days, followed by administration of the mTOR inhibiting compound alone for at least 3 consecutive days - in this case, the duration of time will be at least 5 days; suitably, during the course of treatment, both compounds will be administered within a specified period for at least 2 consecutive days, followed by administration of the mTOR inhibiting compound alone for at least 4 consecutive days - in this case, the duration of time will be at least 6 days; suitably, during the course of treatment, both compounds will be administered within a specified period for at least 2 consecutive days, followed by administration of the mTOR inhibiting compound alone for at least 5 consecutive days - in this case, the duration of time will be at least 7 days; suitably, during the course of treatment, both compounds will be administered within a specified period for at least 2 consecutive days, followed by administration of the mTOR inhibiting compound alone for at least 6 consecutive days - in this case, the duration of time will be at least 8 days; suitably, during the course of treatment, both compounds will be administered within a specified period for at least 2 consecutive days, followed by administration of the mTOR inhibiting compound alone for at least 7 consecutive days - in this case, the duration of time will be at least 9 days; suitably, during the course of treatment, both compounds will be administered within a specified period for at least 3 consecutive days, followed by administration of the mTOR inhibiting compound alone for at least 1 day - in this case, the duration of time will be at least 4 days; suitably, during the course of treatment, both compounds will be administered within a

specified period for at least 3 consecutive days, followed by administration of the mTOR inhibiting compound alone for at least 2 consecutive days - in this case, the duration of time will be at least 5 days; suitably, during the course of treatment, both compounds will be administered within a specified period for at least 3 consecutive days, followed by administration of the mTOR inhibiting compound alone for at least 3 consecutive days - in this case, the duration of time will be at least 6 days; suitably, during the course of treatment, both compounds will be administered within a specified period for at least 3 consecutive days, followed by administration of the mTOR inhibiting compound alone for at least 4 consecutive days - in this case, the duration of time will be at least 7 days; suitably, during the course of treatment, both compounds will be administered within a specified period for at least 3 consecutive days, followed by administration of the mTOR inhibiting compound alone for at least 5 consecutive days - in this case, the duration of time will be at least 8 days; suitably, during the course of treatment, both compounds will be administered within a specified period for at least 3 consecutive days, followed by administration of the mTOR inhibiting compound alone for at least 6 consecutive days - in this case, the duration of time will be at least 9 days; suitably, during the course of treatment, both compounds will be administered within a specified period for at least 3 consecutive days, followed by administration of the mTOR inhibiting compound alone for at least 7 consecutive days - in this case, the duration of time will be at least 10 days; suitably, during the course of treatment, both compounds will be administered within a specified period for at least 4 consecutive days, followed by administration of the mTOR inhibiting compound alone for at least 1 day - in this case, the duration of time will be at least 5 consecutive days; suitably, during the course of treatment, both compounds will be administered within a specified period for at least 4 consecutive days, followed by administration of the TOR inhibiting compound alone for at least 2 consecutive days - in this case, the duration of time will be at least 6 consecutive days; suitably, during the course of treatment, both compounds will be administered within a specified period for at least 4 consecutive days, followed by administration of the mTOR inhibiting compound alone for at least 3 consecutive days - in this case, the duration of time will be at least 7 consecutive days; suitably, during the course of treatment, both compounds will be administered within a specified period for at least 4 consecutive days, followed by administration of the mTOR inhibiting compound alone for at least 4 consecutive days - in this case, the duration of time will be at least 8 consecutive days; suitably, during the course of treatment, both compounds will be administered within a specified period for at least 4 consecutive days, followed by administration of the mTOR inhibiting compound alone for at least 7 consecutive days - in this case, the duration of time will be at least 11 consecutive days; suitably, during the course of treatment, both compounds will be administered within a specified period for at least 5 consecutive days, followed by administration of the mTOR inhibiting compound alone for at least 1 day - in this case, the duration of time will be at least 6 consecutive days; suitably, during the course of treatment, both compounds will be administered within a specified period for at least 5 consecutive days, followed by administration of the mTOR inhibiting compound alone for at least 2 consecutive days - in this case, the duration of time will be at least 7 consecutive days; suitably, during the course of treatment, both compounds will be administered within a specified period for at least 5 consecutive days, followed by administration of the mTOR inhibiting compound alone for at least 3 consecutive days - in this case, the duration of time will be at least 8 consecutive days; suitably, during the course of treatment, both compounds will be administered within a specified period for at least 5 consecutive days, followed by administration of the mTOR inhibiting compound alone for at least 4 consecutive days - in this case, the duration of time will be at least 9 consecutive days; suitably, during the course of treatment, both compounds will be administered within a specified period for at least 5 consecutive days, followed by administration of the mTOR inhibiting compound alone for at least 5 consecutive days - in this case, the duration of time will be at least 10 consecutive days; suitably, during the course of treatment, both compounds will be administered within a specified period for at least 7 consecutive days, followed by administration of the mTOR inhibiting compound alone for at least 2 consecutive days - in this case, the duration of time will be at least 9 consecutive days; suitably, during the course of treatment, both compounds will be administered within a specified period for at least 14 consecutive days, followed by administration of the mTOR inhibiting compound alone for at least 7 consecutive days - in this case, the duration of time will be at least 21 consecutive days; suitably, during the course of treatment, both compounds will be administered within a specified period for at least 30 consecutive days, followed by administration of the mTOR inhibiting compound alone for at least 7 consecutive days - in this case, the duration of time will be at least 37 consecutive days. Suitably, during the course of treatment, both compounds will be administered within a specified period for from 1 to 3 consecutive days, followed by administration of the mTOR inhibiting compound alone for from 3 to 7 consecutive days. Suitably, during the course of treatment, both compounds will be administered within a specified period for from 3 to 6 consecutive days, followed by administration of the mTOR inhibiting compound alone for from 1 to 4 consecutive days. Suitably, during the course of treatment, both compounds will be administered within a specified period for 5 consecutive days, followed by administration of the mTOR inhibiting compound alone for 2 consecutive days. Suitably, during the course of treatment, both compounds will be administered within a specified period for 2 consecutive days, followed by administration of the mTOR inhibiting compound alone for from 3 to 7 consecutive days.

Further regarding "specified period" administration:

Suitably, during the course of treatment, both compounds will be administered within a specified period for at least 1 day, followed by the administration of Compound $C^2$ alone for at least 1 day - in this case, the duration of time will be at least 2 days; suitably, during the course of treatment, both compounds will be administered within a specified period for at least 1 day, followed by administration of Compound $C^2$ alone for at least 2 days - in this case, the duration of time will be at least 3 days; suitably, during the course of treatment, both compounds will be administered within a specified period for at least 1 day, followed by administration of Compound $C^2$ alone for at least 3 days - in this case, the duration of time will be at least 4 days; suitably, during the course of treatment, both compounds will be administered within a specified period for at least 1 day, followed by administration of Compound $C^2$ alone for at least 4 days - in this case, the duration of time will be at least 5 days; suitably, during the course of treatment, both compounds will be administered within a specified period for at least 1 day, followed by administration of Compound $C^2$ alone for at least 5 days - in this case, the duration of time will be at least 6 days; suitably, during the course of treatment, both compounds will be administered within a specified period for at least 1 day, followed by administration of Compound $C^2$ alone for at least 6 days - in this case, the duration of time will be at least 7 days; suitably, during the course of treatment, both compounds will be administered within a specified period for at least 1 day, followed by administration of Compound $C^2$ alone for at least 7 days - in this case, the duration of time will be at least 8 days; suitably, during the course of treatment, both compounds will be administered within a specified period for at least 2 consecutive days, followed by administration of Compound $C^2$ alone for at least 1 day - in this case, the duration of time will be at least 3 days; suitably, during the course of treatment, both compounds will be administered within a specified period for at least 2 consecutive days, followed by administration of Compound $C^2$ alone for at least 2 consecutive days - in this case, the duration of time will be at least 4 days; suitably, during the course of treatment, both compounds will be administered within a specified period for at least 2 consecutive days, followed by administration of Compound $C^2$ alone for at least 3 consecutive days - in this case, the duration of time will be at least 5 days; suitably, during the course of treatment, both compounds will be administered within a specified period for at least 2 consecutive days, followed by administration of Compound $C^2$ alone for at least 4 consecutive days - in this case, the duration of time will be at least 6 days; suitably, during the course of treatment, both compounds will be administered within a specified period for at least 2 consecutive days, followed by administration of Compound $C^2$ alone for at least 5 consecutive days - in this case, the duration of time will be at least 7 days; suitably, during the course of treatment, both compounds will be administered within a specified period for at least 2 consecutive days, followed by administration of Compound $C^2$ alone for at least 6 consecutive days - in this case, the duration of time will be at least 8 days; suitably, during the course of treatment, both compounds will be administered within a specified period for at least 2 consecutive days, followed by administration of Compound $C^2$ alone for at least 7 consecutive days - in this case, the duration of time will be at least 9 days; suitably, during the course of treatment, both compounds will be administered within a specified period for at least 3 consecutive days, followed by administration of Compound $C^2$ alone for at least 1 day - in this case, the duration of time will be at least 4 days; suitably, during the course of treatment, both compounds will be administered within a specified period for at least 3 consecutive days, followed by administration of Compound $C^2$ alone for at least 2 consecutive days - in this case, the duration of time will be at least 5 days; suitably, during the course of treatment, both compounds will be administered within a specified period for at least 3 consecutive days, followed by administration of Compound $C^2$ alone for at least 3 consecutive days - in this case, the duration of time will be at least 6 days; suitably, during the course of treatment, both compounds will be administered within a specified period for at least 3 consecutive days, followed by administration of Compound $C^2$ alone for at least 4 consecutive days - in this case, the duration of time will be at least 7 days; suitably, during the course of treatment, both compounds will be administered within a specified period for at least 3 consecutive days, followed by administration of Compound $C^2$ alone for at least 5 consecutive days - in this case, the duration of time will be at least 8 days; suitably, during the course of treatment, both compounds will be administered within a specified period for at least 3 consecutive days, followed by administration of Compound $C^2$ alone for at least 6 consecutive days - in this case, the duration of time will be at least 9 days; suitably, during the course of treatment, both compounds will be administered within a specified period for at least 3 consecutive days, followed by administration of Compound $B^2$ alone for at least 7 consecutive days - in this case, the duration of time will be at least 10 days; suitably, during the course of treatment, both compounds will be administered within a specified period for at least 4 consecutive days, followed by administration of Compound $C^2$ alone for at least 1 day - in this case, the duration of time will be at least 5 consecutive days; suitably, during the course of treatment, both compounds will be administered within a specified period for at least 4 consecutive days, followed by administration of Compound $C^2$ alone for at least 2 consecutive days - in this case, the duration of time will be at least 6 consecutive days; suitably, during the course of treatment, both compounds will be administered within a specified period for at least 4 consecutive days, followed by administration of Compound $C^2$ alone for at least 3 consecutive days - in this case, the duration of time will be at least 7 consecutive days; suitably, during the course of treatment, both compounds

will be administered within a specified period for at least 4 consecutive days, followed by administration of Compound $C^2$ alone for at least 4 consecutive days - in this case, the duration of time will be at least 8 consecutive days; suitably, during the course of treatment, both compounds will be administered within a specified period for at least 4 consecutive days, followed by administration of Compound $C^2$ alone for at least 7 consecutive days - in this case, the duration of time will be at least 11 consecutive days; suitably, during the course of treatment, both compounds will be administered within a specified period for at least 5 consecutive days, followed by administration of Compound $C^2$ alone for at least 1 day - in this case, the duration of time will be at least 6 consecutive days; suitably, during the course of treatment, both compounds will be administered within a specified period for at least 5 consecutive days, followed by administration of Compound $C^2$ alone for at least 2 consecutive days - in this case, the duration of time will be at least 7 consecutive days; suitably, during the course of treatment, both compounds will be administered within a specified period for at least 5 consecutive days, followed by administration of Compound $C^2$ alone for at least 3 consecutive days - in this case, the duration of time will be at least 8 consecutive days; suitably, during the course of treatment, both compounds will be administered within a specified period for at least 5 consecutive days, followed by administration of Compound $C^2$ alone for at least 4 consecutive days - in this case, the duration of time will be at least 9 consecutive days; suitably, during the course of treatment, both compounds will be administered within a specified period for at least 5 consecutive days, followed by administration of Compound $C^2$ alone for at least 5 consecutive days - in this case, the duration of time will be at least 10 consecutive days; suitably, during the course of treatment, both compounds will be administered within a specified period for at least 7 consecutive days, followed by administration of Compound $C^2$ alone for at least 2 consecutive days - in this case, the duration of time will be at least 9 consecutive days; suitably, during the course of treatment, both compounds will be administered within a specified period for at least 14 consecutive days, followed by administration of Compound $C^2$ alone for at least 7 consecutive days - in this case, the duration of time will be at least 21 consecutive days; suitably, during the course of treatment, both compounds will be administered within a specified period for at least 30 consecutive days, followed by administration of Compound $C^2$ alone for at least 7 consecutive days - in this case, the duration of time will be at least 37 consecutive days. Suitably, during the course of treatment, both compounds will be administered within a specified period for from 1 to 3 consecutive days, followed by administration of Compound $C^2$ alone for from 3 to 7 consecutive days. Suitably, during the course of treatment, both compounds will be administered within a specified period for from 3 to 6 consecutive days, followed by administration of Compound $C^2$ alone for from 1 to 4 consecutive days. Suitably, during the course of treatment, both compounds will be administered within a specified period for 5 consecutive days, followed by administration of Compound $C^2$ alone for 2 consecutive days. Suitably, during the course of treatment, both compounds will be administered within a specified period for 2 consecutive days, followed by administration of Compound $C^2$ alone for from 3 to 7 consecutive days.

Further regarding "specified period" administration:

Suitably, during the course of treatment, the mTOR inhibiting compound and Compound $C^2$ will be administered within a specified period for from 1 to 3 days during a 7 day period, and during the other days of the 7 day period the mTOR inhibiting compound will be administered alone. Suitably, this 7 day protocol is repeated for 2 cycles or for 14 days; suitably for 4 cycles or 28 days; suitably for continuous administration.

Suitably, during the course of treatment, the mTOR inhibiting compound and Compound $C^2$ will be administered within a specified period for from 1 to 3 days during a 7 day period, and during the other days of the 7 day period Compound $C^2$ will be administered alone. Suitably, this 7 day protocol is repeated for 2 cycles or for 14 days; suitably for 4 cycles or 28 days; suitably for continuous administration.

Suitably, during the course of treatment, the mTOR inhibiting compound and Compound $C^2$ will be administered within a specified period for 3 days during a 7 day period, and during the other days of the 7 day period the mTOR inhibiting compound will be administered alone. Suitably, this 7 day protocol is repeated for 2 cycles or for 14 days; suitably for 4 cycles or 28 days; suitably for continuous administration.

Suitably, during the course of treatment, the mTOR inhibiting compound and Compound $C^2$ will be administered within a specified period for 3 days during a 7 day period, and during the other days of the 7 day period Compound $C^2$ will be administered alone. Suitably, this 7 day protocol is repeated for 2 cycles or for 14 days; suitably for 4 cycles or 28 days; suitably for continuous administration.

Suitably, during the course of treatment, the mTOR inhibiting compound and Compound $C^2$ will be administered within a specified period for 2 days during a 7 day period, and during the other days of the 7 day period the mTOR inhibiting compound will be administered alone. Suitably, this 7 day protocol is repeated for 2 cycles or for 14 days;

suitably for 4 cycles or 28 days; suitably for continuous administration.

Suitably, during the course of treatment, the mTOR inhibiting compound and Compound $C^2$ will be administered within a specified period for 2 days during a 7 day period, and during the other days of the 7 day period Compound $C^2$ will be administered alone. Suitably, this 7 day protocol is repeated for 2 cycles or for 14 days; suitably for 4 cycles or 28 days; suitably for continuous administration.

Suitably, during the course of treatment, the mTOR inhibiting compound and Compound $C^2$ will be administered within a specified period for 1 day during a 7 day period, and during the other days of the 7 day period the mTOR inhibiting compound will be administered alone. Suitably, this 7 day protocol is repeated for 2 cycles or for 14 days; suitably for 4 cycles or 28 days; suitably for continuous administration.

Suitably, during the course of treatment, the mTOR inhibiting compound and Compound $C^2$ will be administered within a specified period for 1 day during a 7 day period, and during the other days of the 7 day period Compound $C^2$ will be administered alone. Suitably, this 7 day protocol is repeated for 2 cycles or for 14 days; suitably for 4 cycles or 28 days; suitably for continuous administration.

Suitably, during the course of treatment, the mTOR inhibiting compound and Compound $C^2$ will be administered within a specified period for from 1 to 5 days during a 14 day period, and during the other days of the 14 day period the mTOR inhibiting compound will be administered alone. Suitably, this 14 day protocol is repeated for 2 cycles or for 28 days; suitably for continuous administration.

Suitably, during the course of treatment, the mTOR inhibiting compound and Compound $C^2$ will be administered within a specified period for from 1 to 5 days during a 14 day period, and during the other days of the 14 day period Compound $C^2$ will be administered alone. Suitably, this 14 day protocol is repeated for 2 cycles or for 28 days; suitably for continuous administration.

Suitably, if the compounds are not administered during a "specified period", they are administered sequentially. By the term "sequential administration", and derivates thereof, as used herein is meant that one of an mTOR inhibiting compound and Compound $C^2$ is administered for one or more consecutive days and the other of an mTOR inhibiting compound and Compound $C^2$ is subsequently administered for one or more consecutive days. Also, contemplated herein is a drug holiday utilized between the sequential administration of one of an mTOR inhibiting compound and Compound $C^2$ and the other of an mTOR inhibiting compound and Compound $C^2$. As used herein, a drug holiday is a period of days after the sequential administration of one of an mTOR inhibiting compound and Compound $C^2$ and before the administration of the other of an mTOR inhibiting compound and Compound $C^2$ where neither an mTOR inhibiting compound nor Compound $C^2$ is administered. Suitably the drug holiday will be a period of days selected from: 1 day, 2 days, 3 days, 4 days, 5 days, 6 days, 7 days, 8 days, 9 days, 10 days, 11 days, 12 days, 13 days and 14 days.

Regarding sequential administration:

Suitably, one of an mTOR inhibiting compound and Compound $C^2$ is administered for from 1 to 30 consecutive days, followed by an optional drug holiday, followed by administration of the other of an mTOR inhibiting compound and Compound $C^2$ for from 1 to 30 consecutive days. Suitably, one of an mTOR inhibiting compound and Compound $C^2$ is administered for from 1 to 21 consecutive days, followed by an optional drug holiday, followed by administration of the other of an mTOR inhibiting compound and Compound $C^2$ for from 1 to 21 consecutive days. Suitably, one of an mTOR inhibiting compound and Compound $C^2$ is administered for from 1 to 14 consecutive days, followed by an optional drug holiday of from 1 to 14 days, followed by administration of the other of an mTOR inhibiting compound and Compound $C^2$ for from 1 to 14 consecutive days. Suitably, one of an mTOR inhibiting compound and Compound $C^2$ is administered for from 2 to 7 consecutive days, followed by an optional drug holiday of from 1 to 10 days, followed by administration of the other of an mTOR inhibiting compound and Compound $C^2$ for from 2 to 7 consecutive days.
Suitably, Compound $C^2$ will be administered first in the sequence, followed by an optional drug holiday, followed by administration of an mTOR inhibiting compound. Suitably, Compound $C^2$ is administered for from 1 to 21 consecutive days, followed by an optional drug holiday, followed by administration of an mTOR inhibiting compound for from 1 to 21 consecutive days. Suitably, Compound $C^2$ is administered for from 3 to 21 consecutive days, followed by a drug holiday of from 1 to 14 days, followed by administration of an mTOR inhibiting compound for from 3 to 21 consecutive days. Suitably, Compound $C^2$ is administered for from 3 to 21 consecutive days, followed by a drug

holiday of from 3 to 14 days, followed by administration of an mTOR inhibiting compound for from 3 to 21 consecutive days. Suitably, Compound $C^2$ is administered for 21 consecutive days, followed by an optional drug holiday, followed by administration of an mTOR inhibiting compound for 14 consecutive days. Suitably, Compound $C^2$ is administered for 14 consecutive days, followed by a drug holiday of from 1 to 14 days, followed by administration of an mTOR inhibiting compound for 14 consecutive days. Suitably, Compound $C^2$ is administered for 7 consecutive days, followed by a drug holiday of from 3 to 10 days, followed by administration of an mTOR inhibiting compound for 7 consecutive days. Suitably, Compound $C^2$ is administered for 3 consecutive days, followed by a drug holiday of from 3 to 14 days, followed by administration of an mTOR inhibiting compound for 7 consecutive days. Suitably, Compound $C^2$ is administered for 3 consecutive days, followed by a drug holiday of from 3 to 10 days, followed by administration of an mTOR inhibiting compound for 3 consecutive days. Suitably, Compound $C^2$ is administered for 7 consecutive days, followed by administration of an mTOR inhibiting compound for 1 day. Suitably, Compound $C^2$ is administered for 6 consecutive days, followed by administration of an mTOR inhibiting compound for 1 day.

Suitably, an mTOR inhibiting compound is administered for 2 consecutive days, followed by administration of Compound $C^2$ for from 3 to 7 consecutive days. Suitably, an mTOR inhibiting compound is administered for 2 consecutive days, followed by administration of Compound $C^2$ for 5 consecutive days.

Suitably, an mTOR inhibiting compound will be administered first in the sequence, followed by an optional drug holiday, followed by administration of Compound $C^2$. Suitably, an mTOR inhibiting compound is administered for from 1 to 21 consecutive days, followed by an optional drug holiday, followed by administration of Compound $C^2$ for from 1 to 21 consecutive days. Suitably, an mTOR inhibiting compound is administered for from 3 to 21 consecutive days, followed by an optional drug holiday of from 1 to 14 days, followed by administration of Compound $C^2$ for from 3 to 21 consecutive days. Suitably, an mTOR inhibiting compound is administered for from 3 to 21 consecutive days, followed by an optional drug holiday of from 3 to 14 days, followed by administration of Compound $C^2$ for from 3 to 21 consecutive days. Suitably, an mTOR inhibiting compound is administered for 21 consecutive days, followed by an optional drug holiday, followed by administration of Compound $C^2$ for 14 consecutive days. Suitably, an mTOR inhibiting compound is administered for 14 consecutive days, followed by an optional drug holiday of from 1 to 14 days, followed by administration of Compound $C^2$ for 14 consecutive days. Suitably, an mTOR inhibiting compound is administered for 7 consecutive days, followed by an optional drug holiday of from 3 to 10 days, followed by administration of Compound $C^2$ for 7 consecutive days. Suitably, an mTOR inhibiting compound is administered for 3 consecutive days, followed by an optional drug holiday of from 3 to 14 days, followed by administration of Compound $C^2$ for 7 consecutive days. Suitably, an mTOR inhibiting compound is administered for 3 consecutive days, followed by an optional drug holiday of from 3 to 10 days, followed by administration of Compound $C^2$ for 3 consecutive days. Suitably, an mTOR inhibiting compound is administered for 7 consecutive days, followed by administration of Compound $C^2$ for 1 day. Suitably, an mTOR inhibiting compound is administered for 6 consecutive days, followed by administration of Compound $C^2$ for 1 day.

Suitably, Compound $C^2$ is administered for 2 consecutive days, followed by administration of an mTOR inhibiting compound for from 3 to 7 consecutive days. Suitably, Compound $C^2$ is administered for 2 consecutive days, followed by administration of an mTOR inhibiting compound for 5 consecutive days.

It is understood that a "specified period" administration and a "sequential" administration can be followed by repeat dosing or can be followed by an alternate dosing protocol, and a drug holiday may precede the repeat dosing or alternate dosing protocol.

Suitably, the amount of Compound $C^2$ administered as part of the combination according to the present invention will be an amount selected from about 0.125mg to about 10mg; suitably, the amount will be selected from about 0.25mg to about 9mg; suitably, the amount will be selected from about 0.25mg to about 8mg; suitably, the amount will be selected from about 0.5mg to about 8mg; suitably, the amount will be selected from about 0.5mg to about 7mg; suitably, the amount will be selected from about 1mg to about 7mg; suitably, the amount will be about 5mg. Accordingly, the amount of Compound $C^2$ administered as part of the combination according to the present invention will be an amount selected from about 0.125mg to about 10 mg. For example, the amount of Compound $C^2$ administered as part of the combination according to the present invention can be 0.125mg, 0.25mg, 0.5mg, 0.75mg, 1mg, 1.5mg, 2mg, 2.5mg, 3mg, 3.5mg, 4mg, 4.5mg, 5mg, 5.5mg, 6mg, 6.5mg, 7mg, 7.5mg, 8mg, 8.5mg, 9mg, 9.5mg, 10mg. Suitably, the selected amount of Compound $C^2$ is administered twice a day. Suitably, the selected amount of Compound $C^2$ is administered once a day. Suitably, the administration of Compound $C^2$ will begin as a loading dose. Suitably, the loading dose will be an amount from 2 to 100 times the maintenance dose; suitably from 2 to 10 times; suitably from 2 to 5 times; suitably 2 times; suitably 3 times; suitably 4 times; suitably 5 times. Suitably, the loading does will be administered from 1 to 7 days; suitably from 1 to 5 days; suitably from 1 to 3 days; suitably for 1 day; suitably for 2 days; suitably for 3 days, followed by a maintenance dosing protocol.

The amount of mTOR inhibitor will depend ultimately on the particular agent used.

Suitably, the amount of everolimus administered as part of the combination according to the present invention

will be an amount selected from about 1.25mg to about 20mg; suitably, the amount will be selected from about 2mg to about 15mg; suitably, the amount will be selected from about 2.5mg to about 10mg. Accordingly, the amount of everolimus administered as part of the combination according to the present invention will be an amount selected from about 1.25mg to about 20mg. For example, the amount of everolimus administered as part of the combination according to the present invention can be 1.25mg, 1.5mg, 2mg, 2.5mg, 3mg, 3.5mg, 4mg, 4.5mg, 5mg, 5.5mg, 6mg, 6.5mg, 7mg, 7.5mg, 8mg, 8.5mg, 9mg, 9.5mg, 10mg, 11mg, 12mg, 13mg, 14mg, 15mg, 16mg, 17mg, 18mg, 19mg, 20mg. Suitably, the selected amount of everolimus is administered twice a day. Suitably, the selected amount of everolimus is administered once a day. Suitably, the administration of everolimus will begin as a loading dose. Suitably, the loading dose will be an amount from 2 to 100 times the maintenance dose; suitably from 2 to 10 times; suitably from 2 to 5 times; suitably 2 times; suitably 3 times; suitably 4 times; suitably 5 times. Suitably, the loading does will be administered from 1 to 7 days; suitably from 1 to 5 days; suitably from 1 to 3 days; suitably for 1 day; suitably for 2 days; suitably for 3 days, followed by a maintenance dosing protocol.

Suitably, the amount of temsirolimus administered as part of the combination according to the present invention will be an amount infused over a 30 to 60 minute period, where the amount is selected from about 5mg to about 50mg; suitably, the amount will be selected from about 10mg to about 40mg; suitably, the amount will be selected from about 15mg to about 35mg. Accordingly, the amount of temsirolimus administered as part of the combination according to the present invention will be an amount selected from about 5mg to about 50mg. For example, the amount of temsirolimus administered as part of the combination according to the present invention can be 5mg, 10mg, 15mg, 20mg, 25mg, 30mg, 35mg, 40mg, 45mg, 50mg. Suitably, the selected amount of temsirolimus is administered twice a day. Suitably, the selected amount of temsirolimus is administered once a day. Suitably, the administration of temsirolimus will begin as a loading dose. Suitably, the loading dose will be an amount from 2 to 100 times the maintenance dose; suitably from 2 to 10 times; suitably from 2 to 5 times; suitably 2 times; suitably 3 times; suitably 4 times; suitably 5 times. Suitably, the loading does will be administered from 1 to 7 days; suitably from 1 to 5 days; suitably from 1 to 3 days; suitably for 1 day; suitably for 2 days; suitably for 3 days, followed by a maintenance dosing protocol.

As used herein, all amounts specified for a compound of the presently invented combinations, suitably Compound $C^2$, are indicated as the amount of free or unsalted, unsolvated compound.

## METHOD OF TREATMENT

The combinations of the invention, are believed to have utility in disorders wherein the inhibition of MEK and/or mTOR is beneficial.

The method of the present invention may also be employed with other therapeutic methods of cancer treatment.

The combination of the invention may be used alone or in combination with one or more other therapeutic agents. The invention thus provides in a further aspect a further combination comprising a combination of the invention with a further therapeutic agent or agents, compositions and medicaments comprising the combination and use of the further combination, compositions and medicaments in therapy, in particular in the treatment of diseases susceptible to inhibition of MEK and/or mTOR.

In the embodiment, the combination of the invention may be employed with other therapeutic methods of cancer treatment. In particular, in anti-neoplastic therapy, combination therapy with other chemotherapeutic, hormonal, antibody agents as well as surgical and/or radiation treatments other than those mentioned above are envisaged. Combination therapies according to the present invention thus include the administration of Compound $C^2$ and an mTOR inhibiting compound as well as optional use of other therapeutic agents including other anti-neoplastic agents. Such combination of agents may be administered together or separately and, when administered separately this may occur simultaneously or sequentially in any order, both close and remote in time. In one embodiment, the pharmaceutical combination includes Compound $C^2$ and an mTOR inhibiting compound, and optionally at least one additional anti-neoplastic agent.

As indicated, therapeutically effective amounts of Compound $C^2$ and an mTOR inhibiting compound are discussed above. The therapeutically effective amount of the further therapeutic agents of the present invention will depend upon a number of factors including, for example, the age and weight of the mammal, the precise condition requiring treatment, the severity of the condition, the nature of the formulation, and the route of administration. Ultimately, the therapeutically effective amount will be at the discretion of the attendant physician or veterinarian. The relative timings of administration will be selected in order to achieve the desired combined therapeutic effect.

In one embodiment, the further anti-cancer therapy is surgical and/or radiotherapy.

In one embodiment, the further anti-cancer therapy is at least one additional anti-neoplastic agent.

Any anti-neoplastic agent that has activity versus a susceptible tumor being treated may be utilized in the combination. Typical anti-neoplastic agents useful include, but are not limited to, anti-microtubule agents such as diterpenoids and vinca alkaloids; platinum coordination complexes; alkylating agents such as nitrogen mustards, oxazaphosphorines, alkylsulfonates, nitrosoureas, and triazenes; antibiotic agents such as anthracyclins, actinomycins and bleomycins; topoisomerase II inhibitors such as epipodophyllotoxins; antimetabolites such as purine and pyrimidine analogues and anti-folate compounds; topoisomerase I inhibitors such as camptothecins; hormones and hormonal analogues; signal transduction pathway inhibitors; non-receptor tyrosine angiogenesis inhibitors; immunotherapeutic agents; proapoptotic agents; and cell cycle signaling inhibitors.

**Anti-microtubule or anti-mitotic agents:**

Anti-microtubule or anti-mitotic agents are phase specific agents active against the microtubules of tumor cells during M or the mitosis phase of the cell cycle. Examples of anti-microtubule agents include, but are not limited to, diterpenoids and vinca alkaloids.

Diterpenoids, which are derived from natural sources, are phase specific anti - cancer agents that operate at the $G_2$/M phases of the cell cycle. It is believed that the diterpenoids stabilize the β-tubulin subunit of the microtubules, by binding with this protein. Disassembly of the protein appears then to be inhibited with mitosis being arrested and cell death following. Examples of diterpenoids include, but are not limited to, paclitaxel and its analog docetaxel.

Paclitaxel, 5β,20-epoxy-1,2α,4,7β,10β,13α-hexa-hydroxytax-11-en-9-one 4,10-diacetate 2-benzoate 13-ester with (2R,3S)-N-benzoyl-3-phenylisoserine; is a natural diterpene product isolated from the Pacific yew tree *Taxus brevifolia* and is commercially available as an injectable solution TAXOL®. It is a member of the taxane family of terpenes. Paclitaxel has been approved for clinical use in the treatment of refractory ovarian cancer in the United States (Markman et al., Yale Journal of Biology and Medicine, 64:583, 1991; McGuire et al., Ann. Intem, Med., 111:273,1989) and for the treatment of breast cancer (Holmes et al., J. Nat. Cancer Inst., 83:1797,1991.) It is a potential candidate for treatment of neoplasms in the skin (Einzig et. al., Proc. Am. Soc. Clin. Oncol., 20:46) and head and neck carcinomas (Forastire et. al., Sem. Oncol., 20:56, 1990). The compound also shows potential for the treatment of polycystic kidney disease (Woo et. al., Nature, 368:750. 1994), lung cancer and malaria. Treatment of patients with paclitaxel results in bone marrow suppression (multiple cell lineages, Ignoff, R.J. et. al, Cancer Chemotherapy Pocket Guide, 1998) related to the duration of dosing above a threshold concentration (50nM) (Kearns, C.M. et. al., Seminars in Oncology, 3(6) p.16-23, 1995).

Docetaxel, (2R,3S)-N-carboxy-3-phenylisoserine,N-*tert*-butyl ester, 13-ester with 5β-20-epoxy-1,2α,4,7β,10β,13α-hexahydroxytax-11-en-9-one 4-acetate 2-benzoate, trihydrate; is commercially available as an injectable solution as TAXOTERE®. Docetaxel is indicated for the treatment of breast cancer. Docetaxel is a semisynthetic derivative of paclitaxel *q.v.,* prepared using a natural precursor, 10-deacetyl-baccatin III, extracted from the needle of the European Yew tree.

Vinca alkaloids are phase specific anti-neoplastic agents derived from the periwinkle plant. Vinca alkaloids act at the M phase (mitosis) of the cell cycle by binding specifically to tubulin. Consequently, the bound tubulin molecule is unable to polymerize into microtubules. Mitosis is believed to be arrested in metaphase with cell death following. Examples of vinca alkaloids include, but are not limited to, vinblastine, vincristine, and vinorelbine.

Vinblastine, vincaleukoblastine sulfate, is commercially available as VELBAN® as an injectable solution. Although, it has possible indication as a second line therapy of various solid tumors, it is primarily indicated in the treatment of testicular cancer and various lymphomas including Hodgkin's Disease; and lymphocytic and histiocytic lymphomas. Myelosuppression is the dose limiting side effect of vinblastine.

Vincristine, vincaleukoblastine, 22-oxo-, sulfate, is commercially available as ONCOVIN® as an injectable solution. Vincristine is indicated for the treatment of acute leukemias and has also found use in treatment regimens for Hodgkin's and non-Hodgkin's malignant lymphomas. Alopecia and neurologic effects are the most common side effect of vincristine and to a lesser extent myelosupression and gastrointestinal mucositis effects occur.

Vinorelbine, 3',4'-didehydro -4'-deoxy-C'-norvincaleukoblastine [R-(R*,R*)-2,3-dihydroxybutanedioate (1:2)(salt)], commercially available as an injectable solution of vinorelbine tartrate (NAVELBINE®), is a semisynthetic vinca alkaloid. Vinorelbine is indicated as a single agent or in combination with other chemotherapeutic agents, such as cisplatin, in the treatment of various solid tumors, particularly non-small cell lung, advanced breast, and hormone refractory prostate cancers. Myelosuppression is the most common dose limiting side effect of vinorelbine.

**Platinum coordination complexes:**

Platinum coordination complexes are non-phase specific anti-cancer agents, which are interactive with DNA. The platinum complexes enter tumor cells, undergo, aquation and form intra- and interstrand crosslinks with DNA causing adverse biological effects to the tumor. Examples of platinum coordination complexes include, but are not limited to, oxaliplatin, cisplatin and carboplatin.

Cisplatin, cis-diamminedichloroplatinum, is commercially available as PLATINOL® as an injectable solution. Cisplatin is primarily indicated in the treatment of metastatic testicular and ovarian cancer and advanced bladder cancer.

Carboplatin, platinum, diammine [1,1-cyclobutane-dicarboxylate(2-)-O,O'], is commercially available as PARAPLATIN® as an injectable solution. Carboplatin is primarily indicated in the first and second line treatment of advanced

ovarian carcinoma.

**Alkylating agents:**

Alkylating agents are non-phase anti-cancer specific agents and strong electrophiles. Typically, alkylating agents form covalent linkages, by alkylation, to DNA through nucleophilic moieties of the DNA molecule such as phosphate, amino, sulfhydryl, hydroxyl, carboxyl, and imidazole groups. Such alkylation disrupts nucleic acid function leading to cell death. Examples of alkylating agents include, but are not limited to, nitrogen mustards such as cyclophosphamide, melphalan, and chlorambucil; alkyl sulfonates such as busulfan; nitrosoureas such as carmustine; and triazenes such as dacarbazine.

Cyclophosphamide, 2-[bis(2-chloroethyl)amino]tetrahydro-2H-1,3,2-oxazaphosphorine 2-oxide monohydrate, is commercially available as an injectable solution or tablets as CYTOXAN®. Cyclophosphamide is indicated as a single agent or in combination with other chemotherapeutic agents, in the treatment of malignant lymphomas, multiple myeloma, and leukemias.

Melphalan, 4-[bis(2-chloroethyl)amino]-L-phenylalanine, is commercially available as an injectable solution or tablets as ALKERAN®. Melphalan is indicated for the palliative treatment of multiple myeloma and non-resectable epithelial carcinoma of the ovary. Bone marrow suppression is the most common dose limiting side effect of melphalan.

Chlorambucil, 4-[bis(2-chloroethyl)amino]benzenebutanoic acid, is commercially available as LEUKERAN® tablets. Chlorambucil is indicated for the palliative treatment of chronic lymphatic leukemia, and malignant lymphomas such as lymphosarcoma, giant follicular lymphoma, and Hodgkin's disease.

Busulfan, 1,4-butanediol dimethanesulfonate, is commercially available as MYLERAN® TABLETS. Busulfan is indicated for the palliative treatment of chronic myelogenous leukemia.

Carmustine, 1,3-[bis(2-chloroethyl)-1-nitrosourea, is commercially available as single vials of lyophilized material as BiCNU®. Carmustine is indicated for the palliative treatment as a single agent or in combination with other agents for brain tumors, multiple myeloma, Hodgkin's disease, and non-Hodgkin's lymphomas.

Dacarbazine, 5-(3,3-dimethyl-1-triazeno)-imidazole-4-carboxamide, is commercially available as single vials of material as DTIC-Dome®. Dacarbazine is indicated for the treatment of metastatic malignant melanoma and in combination with other agents for the second line treatment of Hodgkin's Disease.

**Antibiotic anti-neoplastics** :

Antibiotic anti-neoplastics are non-phase specific agents, which bind or intercalate with DNA. Typically, such action results in stable DNA complexes or strand breakage, which disrupts ordinary function of the nucleic acids leading to cell death. Examples of antibiotic anti-neoplastic agents include, but are not limited to, actinomycins such as dactinomycin, anthrocyclins such as daunorubicin and doxorubicin; and bleomycins.

Dactinomycin, also know as Actinomycin D, is commercially available in injectable form as COSMEGEN®. Dactinomycin is indicated for the treatment of Wilm's tumor and rhabdomyosarcoma.

Daunorubicin, (8S-cis-)-8-acetyl-10-[(3-amino-2,3,6-trideoxy-α-L-lyxo-hexopyranosyl)oxy]-7,8,9,10-tetrahydro-6,8,11-trihydroxy-1-methoxy-5,12 naphthacenedione hydrochloride, is commercially available as a liposomal injectable form as DAUNOXOME® or as an injectable as CERUBIDINE®. Daunorubicin is indicated for remission induction in the treatment of acute nonlymphocytic leukemia and advanced HIV associated Kaposi's sarcoma.

Doxorubicin, (8S, 10S)-10-[(3-amino-2,3,6-trideoxy-α-L-lyxo-hexopyranosyl)oxy]-8-glycoloyl, 7,8,9,10-tetrahydro-6,8,11-trihydroxy-1-methoxy-5,12 naphthacenedione hydrochloride, is commercially available as an injectable form as RUBEX® or ADRIAMYCIN RDF®. Doxorubicin is primarily indicated for the treatment of acute lymphoblastic leukemia and acute myeloblastic leukemia, but is also a useful component in the treatment of some solid tumors and lymphomas.

Bleomycin, a mixture of cytotoxic glycopeptide antibiotics isolated from a strain of *Streptomyces verticillus,* is commercially available as BLENOXANE®. Bleomycin is indicated as a palliative treatment, as a single agent or in combination with other agents, of squamous cell carcinoma, lymphomas, and testicular carcinomas.

**Topoisomerase II inhibitors:**

Topoisomerase II inhibitors include, but are not limited to, epipodophyllotoxins.

Epipodophyllotoxins are phase specific anti-neoplastic agents derived from the mandrake plant. Epipodophyllotoxins typically affect cells in the S and $G_2$ phases of the cell cycle by forming a ternary complex with topoisomerase II and DNA causing DNA strand breaks. The strand breaks accumulate and cell death follows. Examples of epipodophyllotoxins include, but are not limited to, etoposide and teniposide.

Etoposide, 4'-demethyl-epipodophyllotoxin 9[4,6-0-(R)-ethylidene-β-D-glucopyranoside], is commercially available as an injectable solution or capsules as VePESID® and is commonly known as VP-16. Etoposide is indicated as

a single agent or in combination with other chemotherapy agents in the treatment of testicular and non-small cell lung cancers.

Teniposide, 4'-demethyl-epipodophyllotoxin 9[4,6-0-(R)-thenylidene-[β-D-glucopyranoside], is commercially available as an injectable solution as VUMON® and is commonly known as VM-26. Teniposide is indicated as a single agent or in combination with other chemotherapy agents in the treatment of acute leukemia in children.

**Antimetabolite neoplastic agents:**

Antimetabolite neoplastic agents are phase specific anti-neoplastic agents that act at S phase (DNA synthesis) of the cell cycle by inhibiting DNA synthesis or by inhibiting purine or pyrimidine base synthesis and thereby limiting DNA synthesis. Consequently, S phase does not proceed and cell death follows. Examples of antimetabolite anti-neoplastic agents include, but are not limited to, fluorouracil, methotrexate, cytarabine, mecaptopurine, thioguanine, and gemcitabine.

5-fluorouracil, 5-fluoro-2,4- (1H,3H) pyrimidinedione, is commercially available as fluorouracil. Administration of 5-fluorouracil leads to inhibition of thymidylate synthesis and is also incorporated into both RNA and DNA. The result typically is cell death. 5-fluorouracil is indicated as a single agent or in combination with other chemotherapy agents in the treatment of carcinomas of the breast, colon, rectum, stomach and pancreas. Other fluoropyrimidine analogs include 5-fluoro deoxyuridine (floxuridine) and 5-fluorodeoxyuridine monophosphate.

Cytarabine, 4-amino-1-β-D-arabinofuranosyl-2(1H)-pyrimidinone, is commercially available as CYTOSAR-U® and is commonly known as Ara-C. It is believed that cytarabine exhibits cell phase specificity at S-phase by inhibiting DNA chain elongation by terminal incorporation of cytarabine into the growing DNA chain. Cytarabine is indicated as a single agent or in combination with other chemotherapy agents in the treatment of acute leukemia. Other cytidine analogs include 5-azacytidine and 2',2'-difluorodeoxycytidine (gemcitabine).

Mercaptopurine, 1,7-dihydro-6H-purine-6-thione monohydrate, is commercially available as PURINETHOL®. Mercaptopurine exhibits cell phase specificity at S-phase by inhibiting DNA synthesis by an as of yet unspecified mechanism. Mercaptopurine is indicated as a single agent or in combination with other chemotherapy agents in the treatment of acute leukemia. A useful mercaptopurine analog is azathioprine.

Thioguanine, 2-amino-1,7-dihydro-6H-purine-6-thione, is commercially available as TABLOID®. Thioguanine exhibits cell phase specificity at S-phase by inhibiting DNA synthesis by an as of yet unspecified mechanism. Thioguanine is indicated as a single agent or in combination with other chemotherapy agents in the treatment of acute leukemia. Other purine analogs include pentostatin, erythrohydroxynonyladenine, fludarabine phosphate, and cladribine.

Gemcitabine, 2'-deoxy-2', 2'-difluorocytidine monohydrochloride (β-isomer), is commercially available as GEMZAR®. Gemcitabine exhibits cell phase specificity at S-phase and by blocking progression of cells through the G1/S boundary. Gemcitabine is indicated in combination with cisplatin in the treatment of locally advanced non-small cell lung cancer and alone in the treatment of locally advanced pancreatic cancer.

Methotrexate, N-[4[[(2,4-diamino-6-pteridinyl) methyl]methylamino] benzoyl]-L-glutamic acid, is commercially available as methotrexate sodium. Methotrexate exhibits cell phase effects specifically at S-phase by inhibiting DNA synthesis, repair and/or replication through the inhibition of dyhydrofolic acid reductase which is required for synthesis of purine nucleotides and thymidylate. Methotrexate is indicated as a single agent or in combination with other chemotherapy agents in the treatment of choriocarcinoma, meningeal leukemia, non-Hodgkin's lymphoma, and carcinomas of the breast, head, neck, ovary and bladder.

**Topoisomerase I inhibitors:**

Camptothecins, including, camptothecin and camptothecin derivatives are available or under development as Topoisomerase I inhibitors. Camptothecins cytotoxic activity is believed to be related to its Topoisomerase I inhibitory activity. Examples of camptothecins include, but are not limited to irinotecan, topotecan, and the various optical forms of 7-(4-methylpiperazino-methylene)-10,11-ethylenedioxy-20-camptothecin described below.

Irinotecan HCl, (4S)-4,11-diethyl-4-hydroxy-9-[(4-piperidinopiperidino) carbonyloxy]-1H-pyrano[3',4',6,7]indolizino[1,2-b]quinoline-3,14(4H,12H)-dione hydrochloride, is commercially available as the injectable solution CAMPTOSAR®. Irinotecan is a derivative of camptothecin which binds, along with its active metabolite SN-38, to the topoisomerase I - DNA complex. It is believed that cytotoxicity occurs as a result of irreparable double strand breaks caused by interaction of the topoisomerase I : DNA : irintecan or SN-38 ternary complex with replication enzymes. Irinotecan is indicated for treatment of metastatic cancer of the colon or rectum.

Topotecan HCl, (S)-10-[(dimethylamino)methyl]-4-ethyl-4,9-dihydroxy-1H-pyrano[3',4',6,7]indolizino[1,2-b]quinoline-3,14-(4H,12H)-dione monohydrochloride, is commercially available as the injectable solution HYCAMTIN®. Topotecan is a derivative of camptothecin which binds to the topoisomerase I - DNA complex and prevents religation of singles strand breaks caused by Topoisomerase I in response to torsional strain of the DNA molecule. Topotecan is

indicated for second line treatment of metastatic carcinoma of the ovary and small cell lung cancer.

**Hormones and hormonal analogues:**

Hormones and hormonal analogues are useful compounds for treating cancers in which there is a relationship between the hormone(s) and growth and/or lack of growth of the cancer. Examples of hormones and hormonal analogues useful in cancer treatment include, but are not limited to, adrenocorticosteroids such as prednisone and prednisolone which are useful in the treatment of malignant lymphoma and acute leukemia in children ; aminoglutethimide and other aromatase inhibitors such as anastrozole, letrazole, vorazole, and exemestane useful in the treatment of adrenocortical carcinoma and hormone dependent breast carcinoma containing estrogen receptors; progestrins such as megestrol acetate useful in the treatment of hormone dependent breast cancer and endometrial carcinoma; estrogens, androgens, and antiandrogens such as flutamide, nilutamide, bicalutamide, cyproterone acetate and 5α-reductases such as finasteride and dutasteride, useful in the treatment of prostatic carcinoma and benign prostatic hypertrophy; anti-estrogens such as tamoxifen, toremifene, raloxifene, droloxifene, iodoxyfene, as well as selective estrogen receptor modulators (SERMS) such those described in U.S. Patent Nos. 5,681,835, 5,877,219, and 6,207,716, useful in the treatment of hormone dependent breast carcinoma and other susceptible cancers; and gonadotropin-releasing hormone (GnRH) and analogues thereof which stimulate the release of leutinizing hormone (LH) and/or follicle stimulating hormone (FSH) for the treatment prostatic carcinoma, for instance, LHRH agonists and antagagonists such as goserelin acetate and luprolide.

**Signal transduction pathway inhibitors:**

Signal transduction pathway inhibitors are those inhibitors, which block or inhibit a chemical process which evokes an intracellular change. As used herein this change is cell proliferation or differentiation. Signal tranduction inhibitors useful in the present invention include inhibitors of receptor tyrosine kinases, non-receptor tyrosine kinases, SH2/SH3domain blockers, serine/threonine kinases, phosphotidyl inositol-3 kinases, myo-inositol signaling, and Ras oncogenes.

Several protein tyrosine kinases catalyse the phosphorylation of specific tyrosyl residues in various proteins involved in the regulation of cell growth. Such protein tyrosine kinases can be broadly classified as receptor or non-receptor kinases.

Receptor tyrosine kinases are transmembrane proteins having an extracellular ligand binding domain, a transmembrane domain, and a tyrosine kinase domain. Receptor tyrosine kinases are involved in the regulation of cell growth and are generally termed growth factor receptors. Inappropriate or uncontrolled activation of many of these kinases, i.e. aberrant kinase growth factor receptor activity, for example by over-expression or mutation, has been shown to result in uncontrolled cell growth. Accordingly, the aberrant activity of such kinases has been linked to malignant tissue growth. Consequently, inhibitors of such kinases could provide cancer treatment methods. Growth factor receptors include, for example, epidermal growth factor receptor (EGFr), platelet derived growth factor receptor (PDGFr), erbB2, erbB4, ret, vascular endothelial growth factor receptor (VEGFr), tyrosine kinase with immunoglobulin-like and epidermal growth factor homology domains (TIE-2), insulin growth factor -I (IGFI) receptor, macrophage colony stimulating factor (cfms), BTK, ckit, cmet, fibroblast growth factor (FGF) receptors, Trk receptors (TrkA, TrkB, and TrkC), ephrin (eph) receptors, and the RET protooncogene. Several inhibitors of growth receptors are under development and include ligand antagonists, antibodies, tyrosine kinase inhibitors and anti-sense oligonucleotides. Growth factor receptors and agents that inhibit growth factor receptor function are described, for instance, in Kath, John C., Exp. Opin. Ther. Patents (2000) 10(6):803-818; Shawver et al DDT Vol 2, No. 2 February 1997; and Lofts, F. J. et al, "Growth factor receptors as targets", New Molecular Targets for Cancer Chemotherapy, ed. Workman, Paul and Kerr, David, CRC press 1994, London.

Tyrosine kinases, which are not growth factor receptor kinases are termed non-receptor tyrosine kinases. Non-receptor tyrosine kinases useful in the present invention, which are targets or potential targets of anti-cancer drugs, include cSrc, Lck, Fyn, Yes, Jak, cAbl, FAK (Focal adhesion kinase), Brutons tyrosine kinase, and Bcr-Abl. Such non-receptor kinases and agents which inhibit non-receptor tyrosine kinase function are described in Sinh, S. and Corey, S.J., (1999) Journal of Hematotherapy and Stem Cell Research 8 (5): 465 - 80; and Bolen, J.B., Brugge, J.S., (1997) Annual review of Immunology. 15: 371-404.

SH2/SH3 domain blockers are agents that disrupt SH2 or SH3 domain binding in a variety of enzymes or adaptor proteins including, PI3-K p85 subunit, Src family kinases, adaptor molecules (Shc, Crk, Nck, Grb2) and Ras-GAP. SH2/SH3 domains as targets for anti-cancer drugs are discussed in Smithgall, T.E. (1995), Journal of Pharmacological and Toxicological Methods. 34(3) 125-32.

Inhibitors of Serine/Threonine Kinases including MAP kinase cascade blockers which include blockers of Raf kinases (rafk), Mitogen or Extracellular Regulated Kinase (MEKs), and Extracellular Regulated Kinases (ERKs); and Protein kinase C family member blockers including blockers of PKCs (alpha, beta, gamma, epsilon, mu, lambda, iota,

zeta). IkB kinase family (IKKa, IKKb), PKB family kinases, akt kinase family members, and TGF beta receptor kinases. Such Serine/Threonine kinases and inhibitors thereof are described in Yamamoto, T., Taya, S., Kaibuchi, K., (1999), Journal of Biochemistry. 126 (5) 799-803; Brodt, P, Samani, A., and Navab, R. (2000), Biochemical Pharmacology, 60. 1101-1107; Massague, J., Weis-Garcia, F. (1996) Cancer Surveys. 27:41-64; Philip, P.A., and Harris, A.L. (1995), Cancer Treatment and Research. 78: 3-27, Lackey, K. et al Bioorganic and Medicinal Chemistry Letters, (10), 2000, 223-226; U.S. Patent No. 6,268,391; and Martinez-lacaci, L., et al, Int. J. Cancer (2000), 88(1), 44-52.

Inhibitors of Phosphotidyl inositol-3 Kinase family members including blockers of PI3-kinase, ATM, DNA-PK, and Ku are also useful in the present invention. Such kinases are discussed in Abraham, R.T. (1996), Current Opinion in Immunology. 8 (3) 412-8; Canman, C.E., Lim, D.S. (1998), Oncogene 17 (25) 3301-3308; Jackson, S.P. (1997), International Journal of Biochemistry and Cell Biology. 29 (7):935-8; and Zhong, H. et al, Cancer res, (2000) 60(6), 1541-1545.

Also useful in the present invention are Myo-inositol signaling inhibitors such as phospholipase C blockers and Myoinositol analogues. Such signal inhibitors are described in Powis, G., and Kozikowski A., (1994) New Molecular Targets for Cancer Chemotherapy ed., Paul Workman and David Kerr, CRC press 1994, London.

Another group of signal transduction pathway inhibitors are inhibitors of Ras Oncogene. Such inhibitors include inhibitors of farnesyltransferase, geranyl-geranyl transferase, and CAAX proteases as well as anti-sense oligonucleotides, ribozymes and immunotherapy. Such inhibitors have been shown to block ras activation in cells containing wild type mutant ras , thereby acting as antiproliferation agents. Ras oncogene inhibition is discussed in Scharovsky, O.G., Rozados, V.R., Gervasoni, S.I. Matar, P. (2000), Journal of Biomedical Science. 7(4) 292-8; Ashby, M.N. (1998), Current Opinion in Lipidology. 9 (2) 99 - 102; and BioChim. Biophys. Acta, (19899) 1423(3):19-30.

As mentioned above, antibody antagonists to receptor kinase ligand binding may also serve as signal transduction inhibitors. This group of signal transduction pathway inhibitors includes the use of humanized antibodies to the extracellular ligand binding domain of receptor tyrosine kinases. For example Imclone C225 EGFR specific antibody (see Green, M.C. et al, Monoclonal Antibody Therapy for Solid Tumors, Cancer Treat. Rev., (2000), 26(4), 269-286); Herceptin ® erbB2 antibody (see Tyrosine Kinase Signalling in Breast cancer:erbB Family Receptor Tyrosine Kinases, Breast cancer Res., 2000, 2(3), 176-183); and 2CB VEGFR2 specific antibody (see Brekken, R.A. et al, Selective Inhibition of VEGFR2 Activity by a monoclonal Anti-VEGF antibody blocks tumor growth in mice, Cancer Res. (2000) 60, 5117-5124).

**Anti-angiogenic agents:**

Anti-angiogenic agents including non-receptorMEKngiogenesis inhibitors may alo be useful. Anti-angiogenic agents such as those which inhibit the effects of vascular edothelial growth factor, (for example the anti-vascular endothelial cell growth factor antibody bevacizumab [Avastin™], and compounds that work by other mechanisms (for example linomide, inhibitors of integrin $\alpha v\beta 3$ function, endostatin and angiostatin);

**Immunotherapeutic agents:**

Agents used in immunotherapeutic regimens may also be useful in combination with the compounds of formula (I). Immunotherapy approaches, including for example ex-vivo and in-vivo approaches to increase the immunogenecity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies

**Proapoptotoc agents:**

Agents used in proapoptotic regimens (e.g., bcl-2 antisense oligonucleotides) may also be used in the combination of the present invention.

**Cell cycle signalling inhibitors**

Cell cycle signalling inhibitors inhibit molecules involved in the control of the cell cycle. A family of protein kinases called cyclin dependent kinases (CDKs) and their interaction with a family of proteins termed cyclins controls progression through the eukaryotic cell cycle. The coordinate activation and inactivation of different cyclin/CDK complexes is necessary for normal progression through the cell cycle. Several inhibitors of cell cycle signalling are under development. For instance, examples of cyclin dependent kinases, including CDK2, CDK4, and CDK6 and inhibitors for the same are described in, for instance, Rosania et al, Exp. Opin. Ther. Patents (2000) 10(2):215-230.

In one embodiment, the combination of the present invention comprises a compound of formula I or a salt or solvate thereof and at least one anti-neoplastic agent selected from anti-microtubule agents, platinum coordination complexes, alkylating agents, antibiotic agents, topoisomerase II inhibitors, antimetabolites, topoisomerase I inhibitors, hormones and hormonal analogues, signal transduction pathway inhibitors, non-receptor tyrosine MEKngiogenesis inhibitors, immunotherapeutic agents, proapoptotic agents, and cell cycle signaling inhibitors.

In one embodiment, the combination of the present invention comprises a compound of formula I or a salt or solvate thereof and at least one anti-neoplastic agent which is an anti-microtubule agent selected from diterpenoids and vinca alkaloids.

In a further embodiment, the at least one anti-neoplastic agent is a diterpenoid.

In a further embodiment, the at least one anti-neoplastic agent is a vinca alkaloid.

In one embodiment, the combination of the present invention comprises a compound of formula I or a salt or solvate thereof and at least one anti-neoplastic agent, which is a platinum coordination complex.

In a further embodiment, the at least one anti-neoplastic agent is paclitaxel, carboplatin, or vinorelbine.

In a further embodiment, the at least one anti-neoplastic agent is carboplatin.

In a further embodiment, the at least one anti-neoplastic agent is vinorelbine.

In a further embodiment, the at least one anti-neoplastic agent is paclitaxel.

In one embodiment, the combination of the present invention comprises a compound of formula I and salts or solvates thereof and at least one anti-neoplastic agent which is a signal transduction pathway inhibitor.

In a further embodiment the signal transduction pathway inhibitor is an inhibitor of a growth factor receptor kinase VEGFR2, TIE2, PDGFR, BTK, erbB2, EGFr, IGFR-1, TrkA, TrkB, TrkC, or c-fms.

In a further embodiment the signal transduction pathway inhibitor is an inhibitor of a serine/threonine kinase rafk, akt, or PKC-zeta.

In a further embodiment the signal transduction pathway inhibitor is an inhibitor of a non- receptor tyrosine kinase selected from the src family of kinases.

In a further embodiment the signal transduction pathway inhibitor is an inhibitor of c-src.

In a further embodiment the signal transduction pathway inhibitor is an inhibitor of Ras oncogene selected from inhibitors of farnesyl transferase and geranylgeranyl transferase.

In a further embodiment the signal transduction pathway inhibitor is an inhibitor of a serine/threonine kinase selected from the group consisting of PI3K.

In a further embodiment the signal transduction pathway inhibitor is a dual EGFr/erbB2 inhibitor, for example N-{3-Chloro-4-[(3-fluorobenzyl) oxy]phenyl}-6-[5-({[2-(methanesulphonyl) ethyl]amino}methyl)-2-furyl]-4-quinazolinamine (structure below):

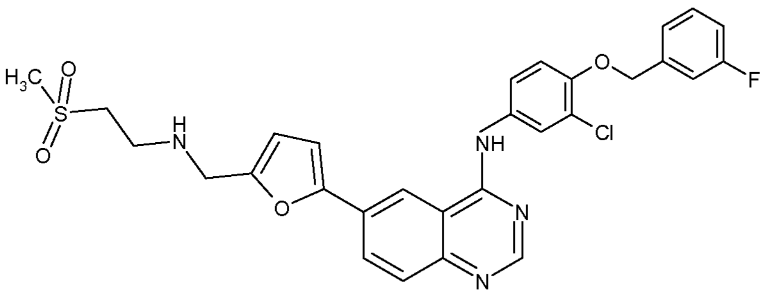

In one embodiment, the combination of the present invention comprises a compound of formula I or a salt or solvate thereof and at least one anti-neoplastic agent which is a cell cycle signaling inhibitor.

In further embodiment, cell cycle signaling inhibitor is an inhibitor of CDK2, CDK4 or CDK6.

In one embodiment the mammal in the methods and uses of the present invention is a human.

While it is possible that, for use in therapy, therapeutically effective amounts of the combinations of the present invention may be administered as the raw chemical, it is preferable to present the combinations as a pharmaceutical composition or compositions. Accordingly, the invention further provides pharmaceutical compositions, which include Compound $C^2$ and/or an mTOR inhibiting compound, and one or more pharmaceutically acceptable carriers. The combinations of the present invention are as described above. The carrier(s) must be acceptable in the sense of being compatible with the other ingredients of the formulation, capable of pharmaceutical formulation, and not deleterious to the recipient thereof. In accordance with another aspect of the invention there is also provided a process for the preparation of a pharmaceutical formulation including admixing Compound $C^2$ and/or an mTOR inhibiting compound with one or more pharmaceutically acceptable carriers. As indicated above, such elements of the pharmaceutical combination utilized may be presented in separate pharmaceutical compositions or formulated together in one pharmaceutical formulation.

Pharmaceutical formulations may be presented in unit dose forms containing a predetermined amount of active ingredient per unit dose. As is known to those skilled in the art, the amount of active ingredient per dose will depend on the condition being treated, the route of administration and the age, weight and condition of the patient. Preferred unit dosage formulations are those containing a daily dose or sub-dose, or an appropriate fraction thereof, of an active ingredient. Furthermore, such pharmaceutical formulations may be prepared by any of the methods well known in the pharmacy art.

Compound $C^2$ and an mTOR inhibiting compound may be administered by any appropriate route. Suitable routes include oral, rectal, nasal, topical (including buccal and sublingual), vaginal, and parenteral (including subcutaneous, intramuscular, intravenous, intradermal, intrathecal, and epidural). It will be appreciated that the preferred route may vary with, for example, the condition of the recipient of the combination and the cancer to be treated. It will also be appreciated that each of the agents administered may be administered by the same or different routes and that Compound $C^2$ and an mTOR inhibiting compound may be compounded together in a pharmaceutical composition/formulation. Suitably, Compound $C^2$ and an mTOR inhibiting compound are administered in separate oral pharmaceutical compositions.

The compounds or combinations of the current invention are incorporated into convenient dosage forms such as capsules, tablets, or injectable preparations. Solid or liquid pharmaceutical carriers are employed. Solid carriers include, starch, lactose, calcium sulfate dihydrate, terra alba, sucrose, talc, gelatin, agar, pectin, acacia, magnesium stearate, and stearic acid. Liquid carriers include syrup, peanut oil, olive oil, saline, and water. Similarly, the carrier may include a prolonged release material, such as glyceryl monostearate or glyceryl distearate, alone or with a wax. The amount of solid carrier varies widely but, preferably, will be from about 25 mg to about 1 g per dosage unit. When a liquid carrier is used, the preparation will suitably be in the form of a syrup, elixir, emulsion, soft gelatin capsule, sterile injectable liquid such as an ampoule, or an aqueous or nonaqueous liquid suspension.

For instance, for oral administration in the form of a tablet or capsule, the active drug component can be combined with an oral, non-toxic pharmaceutically acceptable inert carrier such as ethanol, glycerol, water and the like. Powders are prepared by comminuting the compound to a suitable fine size and mixing with a similarly comminuted pharmaceutical carrier such as an edible carbohydrate, as, for example, starch or mannitol. Flavoring, preservative, dispersing and coloring agent can also be present.

It should be understood that in addition to the ingredients mentioned above, the formulations may include other agents conventional in the art having regard to the type of formulation in question, for example those suitable for oral administration may include flavoring agents.

As indicated, therapeutically effective amounts of the combinations of the invention (Compound $C^2$ in combination with an mTOR inhibiting compound) are administered to a human. Typically, the therapeutically effective amount of the administered agents of the present invention will depend upon a number of factors including, for example, the age and weight of the subject, the precise condition requiring treatment, the severity of the condition, the nature of the formulation, and the route of administration. Ultimately, the therapeutically effective amount will be at the discretion of the attendant physician.

The combinations of the present invention are tested for efficacy, advantageous and synergistic properties according to known procedures.

Suitably, the combinations of the invention are tested for efficacy, advantageous and synergistic properties generally according to the following combination cell proliferation assays. Cells are plated in 384-well plates at 500 cells/well in culture media appropriate for each cell type, supplemented with 10% FBS and 1% penicillin/streptomycin, and incubated overnight at 37°C, 5% $CO_2$. Cells are treated in a grid manner with dilution of Compound $A^2$ (20 dilutions, including no compound, of 2-fold dilutions starting from 1-20 $\mu$M depending of compound) from left to right on 384-well plate and also treated with Compound $B^2$ (20 dilutions, including no compound, of 2-fold dilutions starting from 1-20 $\mu$M depending of compound) from top to bottom on 384-well plate and incubated as above for a further 72 hours. In some instances compounds are added in a staggered manner and incubation time can be extended up to 7days. Cell growth is measured using CellTiter-Glo® reagent according to the manufacturer's protocol and signals are read on a PerkinElmer EnVision™ reader set for luminescence mode with a 0.5-second read. Data are analyzed as described below.

Results are expressed as a percentage of the t=0 value and plotted against compound(s) concentration. The t=0 value is normalized to 100% and represents the number of cells present at the time of compound addition. The cellular response is determined for each compound and/or compound combination using a 4- or 6-parameter curve fit of cell viability against concentration using the IDBS XLfit plug-in for Microsoft Excel software and determining the concentration required for 50% inhibition of cell growth ($gIC_{50}$). Background correction is made by subtraction of values from wells containing no cells. For each drug combination a Combination Index (CI), Excess Over Highest Single Agent (EOHSA) and Excess Over Bliss (EOBliss) are calculated according to known methods such as described in Chou and Talalay (1984) Advances in Enzyme Regulation, 22, 37 to 55; and Berenbaum, MC (1981) Adv. Cancer Research, 35, 269-335.

**Assay 1**

In Assay 1, and corresponding Figures 1 and 2, the MEK inhibiting compound used is Compound A, as defined herein, and the mTOR inhibiting compound used is everolimus, which has the structure indicated below and which is Compound B in Assay 1.

Everolimus:

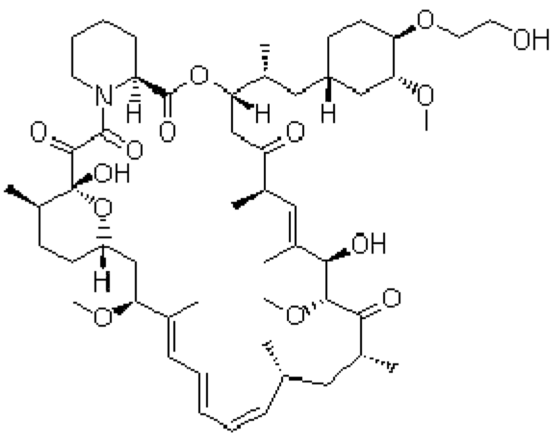

**In vitro cell growth inhibition and apoptosis induction by Compound A & Compound B (everolimus) in a panel lung tumor cell lines**

**Methods**

**Cell lines and growth conditions**

Human tumor cell lines from lung cancer, A427, A549, Calu1, Calu3, Calu6, COR-L23, HOP62, MV522, NCI-H1155, NCI-H1299, NCI-H1355, NCI-H1395, NCI-H157, NCI-H1573, NCI-H1666, NCI-1755, NCI-H1792, NCI-2009, NCI-H2030, NCI-H2122, NCI-H2291, NCI-H23, NCI-H2347, NCI-H358, NCI-H441, NCI-H460, NCI-H650, NCI-H727, SW1573 and SW900 were cultured in RPMI 1640 medium containing 10 % fetal bovine serum (FBS). Cell line mutation data was collated for the status for the RAS and RAF genes. The data source is the cancer cell line mutation screening data published as part of the Catolog of Somatic Mutations in Cancer database (COSMIC) (Bamford S. et al. Br. J. Cancer. 2004. 91:355-58) and/or in house DNA sequencing.

**Cell growth inhibition assay and combination data analysis.**

Cells were seeded in a 96-well tissue culture plate (NUNC 136102) of RPMI medium containing 10% FBS at 500-2,000 cells per well. Approximately 24 hours after plating, cells were exposed to ten, two-fold or three-fold serial dilutions of either Compound A or B or the combination of the two agents at a 10:1 molar ratio (Compounds A and B respectively). The final dosing concentration range for Compound A was 2 -1000 nM and was 0.2 - 100 nM for Compound B. Cells were incubated in the presence of compounds for 3 days. ATP levels were determined by adding Cell Titer Glo® (Promega) according to the manufacturer's protocol. Briefly, Cell Titer Glo® was added to each plate, incubated for 20 minutes then luminescent signal was read on the SpectraMax L plate reader with a 0.5 sec integration time. All assays were run at least in duplicate.

Inhibition of cell growth was estimated after treatment with compound or combination of compounds for three days and comparing the signal to cells treated with vehicle (DMSO). Cell growth was calculated relative to vehicle (DMSO) treated control wells. Concentration of compound that inhibits 50% of control cell growth ($IC_{50}$) was back-interpolated when y = 50% of DMSO treated control wells using nonlinear regression with the equation:

$$y = \frac{A + (B - A)}{1 + \left(\frac{C}{x}\right)^{D}}$$

where A is the minimum response ($y_{min}$), *B* is the maximum response ($y_{max}$), *C* is the inflection point of the curve ($EC_{50}$) and *D* is the Hill coefficient.

Combination effects on potency were evaluated using Combination Index (CI) which was calculated with the back-interpolated $IC_{50}$ values and the mutually non-exclusive equation derived by Chou and Talalay (Chou TC, Talalay P. Adv Enzyme Regul.1984;22:27-55):

$$CI = Da/IC_{50}(a) + Db/IC_{50}(b) + (Da \times Db)/(IC_{50}(a) \times IC_{50}(b))$$

where $IC_{50}(a)$ is the $IC_{50}$ of Compound A; $IC_{50}(b)$ is the $IC_{50}$ for Compound B; Da is the concentration of Compound A in combination with Compound B that inhibited 50% of cell growth; and Db is the concentration of Compound B in combination with Compound A that inhibited 50% of cell growth. In general, a CI value <0.9, between 0.9 and 1.1, or >1.1 indicates synergy, additivity and antagonism, respectively. In general, the smaller the CI number, the greater is the strength of synergy.

The combination effects on the response scale were quantified by EOHSA and Excess Over Highest Single Agent at Total Dose (EOHSATD). The latter is based on the concept of nonlinear blending as was previously described in detail (Peterson & Novick SJ. J Recept Signal Transduct Res 2007. 27:125-46; Peterson J. Frontiers of Bioscience S2, 483-503. 2010). In this study, EOHSA and EOHSATD values are defined as increases in improvement [measured as 'percentage points' (ppts) difference] produced by the combination over the best single drug at its component dose level and at the same total dose as for the combination, respectively. Hence EOHSATD is a stronger "synergy" measure than EOHSA which compares the combination to its (single drug) component doses, rather than to the total dose. Specific methods for calculating EOHSA are previously described. For a given combination (at total dose D), EOHSATD synergy is achieved if the mean response for the combination at total dose D is significantly better than either drug 1 at dose D or drug 2 at dose D. As for EOHSA comparisons, EOHSATD comparisons were conducted using fitted dose response curves at fixed-dose-ratio and the single compound curves. Interactions between Compounds A & B were considered synergistic when EOHSATD > 0.

In this study, co-administration of Compounds A & B exhibit a synergistic interaction in a specific cell line to potency or on the response scale, if the CI <0.9 or the EOHSATD >0 ppt.

**Cell apoptosis assay- caspase-3/7 activation**

For investigation of the induction of apoptosis, cell lines were plated at 5,000 cells per well in a 96-well tissue culture plate and allowed to attach for approximately 24 hours. Cells were then treated with compounds as described above. 24 hours after compound treatment, the levels of active caspase 3 and caspase 7 were determined with the Caspase Glo™ 3/7 (Promega, cat G8093) according to the instructions provided by the manufacturer.

**Results**

**Effects of cell growth inhibition and apoptosis on lung tumor cell lines by Compound A and Compound B combination.**

The effect of cell growth inhibition by a mitogen activated protein/ERK-kinase (MEK) inhibitor Compound A, an mTOR inhibitor Compound B (everolimus) and their combination was determined in a panel of 29 human lung tumor cell lines. The mean $IC_{50}$s (from at least two independent experiments) and the combination effects at $IC_{50}$s are summarized in Table 1 with RAS and RAF mutation status.

Referring to Table 1, 17 out of 29 lines displayed sensitivity to Compound A with $IC_{50}$s < 100 nM, whereas 2 out 29 lines were sensitive to everolimus ($IC_{50}$s < 100 nM). The combination of Compound A and Compound B was synergistic with CI values of 0.19 and 0.62 in A549 and H2122 lines respectively and with EOHSATD values between 4 and 25 ppts in 27 out of 29 lines. In addition, the combination of Compound A and Compound B also showed enhancement of cell growth inhibition with EOHSA values between 5 and 40 ppts in all 29 lines. Note, CI values could not be calculated therefore not applicable where the single agent values were outside of the range tested. Of interest, the

combined administration of Compound A and Compound B in the lung tumor lines showed synergistic effect demonstrated by the CI values <0.9, and EOHSATD >0, or resulted in a much reduced IC50 values (1-22 nM for Compound A and 0.1-2 nM for Compound B in 25 out of 29 lines) comparing to that of Compound A or Compound B, administered alone, where at least one of the single agents did not result in 50% inhibition within the tested range. Representative dose response curves of cell growth inhibition by Compound A and Compound B single agents and their combination are shown for A427, A549, Calu6 and H2122 cell lines in Figure 1.

These lung tumor lines were further evaluated for the ability of Compound A, Compound B or the combination of Compound A and Compound B to induce apoptosis as determined by caspase 3/7 activities. Activation of caspase 3 is a hallmark of induction of apoptosis. Cell lines A427, A549, Calu6, H2122, H1755, H2347, H727 and SW900 showed enhancement of apoptosis by combination treatment with Compound A and Compound B relative to single agent treatment with Compound A or Compound B. Representative caspase 3/7 activity curves for A427, A549, Calu6 and H2122 cell lines are provided in Figure 2.

Table 1. Cell growth inhibition by Compound A, Compound B and their combination in human lung tumor cell lines.

| Cell lines | RAS/RAF mutation status | $IC_{50}$ values in micromolar (mean stud) | | | | Combination effects at $IC_{50}$ | |
|---|---|---|---|---|---|---|---|
| | | Single Agent | | Combination A:B =10:1 molar ratio | | EOHSAT D (ppt) | EOHSA (ppt) |
| | | Compound A | Compound B | Compound A | Compound B | | |
| MV522 | BRAF_V600E | 0.001 ± 0.000 | >0.1 | 0.001 ± 0.000 | 0.0001 ± 0.0000 | NA | 5 ± 1 |
| NCI-H727 | KRAS_G12V | 0.003 ± 0.001 | >0.1 | 0.002 ± 0.000 | 0.0002 ± 0.0000 | 9 ± 8 | 12 ± 8 |
| NCI-H650 | KRAS_Q61L | 0.004 ± 0.002 | >0.1 | 0.002 ± 0.001 | 0.0002 ± 0.0001 | 20 ± 0 | 23 ± 0 |
| NCI-H2291 | KRAS_G12F | 0.004 ± 0.000 | >0.1 | 0.002 ± 0.001 | 0.0002 ± 0.0001 | 11 ± 5 | 14 ± 5 |
| NCI-H2122* | KRAS_G12C | 0.005 ± 0.001 | 0.097 ± 0.037 | 0.003 ± 0.000 | 0.0003 ± 0.0000 | 13 ± 5 | 15 ± 5 |
| Calu6 | KRAS_Q61K | 0.005 ± 0.000 | >0.1 | 0.003 ± 0.001 | 0.0003 ± 0.0001 | 9 ± 3 | 11 ± 3 |
| NCI-H2347 | RAS_H,N_Q61R | 0.007 ± 0.002 | >0.1 | 0.002 ± 0.000 | 0.0002 ± 0.0000 | 10 ± 4 | 17 ± 4 |
| NCI-H23 | KRAS_G12C | 0.031 ± 0.002 | >0.1 | 0.005 ± 0.001 | 0.0005 ± 0.0001 | 18 ± 4 | 18 ± 2 |
| NCI-H358 | KRAS_G12C | 0.031 ± 0.010 | >0.1 | 0.008 ± 0.001 | 0.0008 ± 0.0001 | 16 ± 0 | 18 ± 2 |
| A549* | KRAS_G12S | 0.032 ± 0.009 | 0.003 ± 0.003 | 0.002 ± 0.000 | 0.0002 ± 0.0000 | NA | 14 ± 27 |
| NCI-H460 | KRAS_Q61H | 0.037 ± 0.011 | >0.07 | 0.002 ± 0.002 | 0.0002 ± 0.0002 | 5 ± 3 | 40 ± 4 |
| NCI-H 1666 | BRAF_G466V | 0.044 ± 0.046 | >0.1 | 0.014 ± 0.008 | 0.0014 ±0.0008 | 8 ± 5 | 10 ± 2 |
| NCI-H 1755 | BRAF_G469A | 0.045 ± 0.006 | >0.1 | 0.006 ± 0.000 | 0.0006 ± 0.0000 | 11 ± 0 | 22 ± 3 |
| NCI-H 1792 | KRAS_G12C | 0.059 ± 0.023 | >0.1 | 0.014 ± 0.002 | 0.0014 ± 0.0002 | 13 ± 1 | 15 ± 2 |
| NCI-H 1355 | KRAS_G13C | 0.067 ± 0.024 | >0.1 | 0.004 ± 0.000 | 0.0004 ± 0.0000 | 7 ± 2 | 16 ± 5 |
| NCI-H 1573 | KRAS-G12A | 0.082 ± 0.011 | >0.1 | 0.012 ± 0.003 | 0.0012 ± 0.0003 | 14 ± 5 | 15 ± 6 |
| A427 | KRAS_G12D | 0.095 ± 0.068 | >0.1 | 0.005 ± 0.001 | 0.0005 ± 0.0001 | 22 ± 5 | 23 ± 4 |
| SW900 | KRAS_G12V | 0.141 ± 0.002 | >0.1 | 0.022 ± 0.002 | 0.0022 ± 0.0002 | 20 ± 5 | 20 ± 2 |
| NCI-H2009 | KRAS-G12A | 0.204 ± 0.078 | >0.1 | 0.020 ± 0.003 | 0.0020 ± 0.0003 | 21 ± 7 | 21 ± 1 |
| NCI-H 1299 | NRAS_Q61K | 0.277 ± 0.092 | >0.1 | 0.015 ± 0.002 | 0.0015 ± 0.0002 | 16 ± 1 | 17 ± 2 |
| CORL23 | KRAS_G12V | 0.989 ± 0.086 | >0.1 | 0.019 ± 0.008 | 0.0019 ±0.0008 | 12 ± 5 | 12 ±17 |
| NCI-H2030 | KRAS_G12C | >1 | >0.01 | 0.006 ± 0.001 | 0.0006 ± 0.0001 | 4 ± 3 | 14 ± 2 |
| NCI-H 157 | KRAS_G12R | >1 | >0.1 | 0.006 ± 0.000 | 0.0006 ± 0.0000 | 12 ± 3 | 16 ± 1 |

(continued)

| Cell lines | RAS/RAF mutation status | $IC_{50}$ values in micromolar (mean stud) | | | | Combination effects at $IC_{50}$ | |
|---|---|---|---|---|---|---|---|
| | | Single Agent | | Combination A:B =10:1 molar ratio | | EOHSAT D (ppt) | EOHSA (ppt) |
| | | Compound A | Compound B | Compound A | Compound B | | |
| NCI-H 1155 | KRAS_Q61H | >1 | >0.1 | 0.012 ± 0.001 | 0.0012 ± 0.0001 | 15 ± 1 | 21 ± 6 |
| HOP62 | KRAS_G12C | >1 | >0.1 | 0.014 ± 0.004 | 0.0014 ± 0.0004 | 25 ± 3 | 25 ± 8 |
| NCI-H 1395 | BRAF_G469A | >1 | >0.1 | 0.085 ± 0.075 | 0.0085 ± 0.0075 | 14 ± 8 | 17 ± 1 |
| NCI-H441 | KRAS_G12V | >1 | >0.1 | 0.177 ± 0.073 | 0.0177 ± 0.0073 | 14 ± # | 24 ± 6 |
| SW1573 | KRAS_G12C | >1 | >0.1 | 0.255 ± 0.350 | 0.0255 ± 0.0350 | 20 ± # | 21 ± 8 |
| Calul | KRAS_G12C | >1 | >0.1 | 0.890 ± 1.226 | 0.0890 ± 0.1226 | 21 ± 5 | 23 ± 5 |

Table key:
$IC_{50}$: the concentration of Compound(s) that reduces cell growth by 50%;
* Combination Index (CI) values, CI in H2122= 0.62 ±0.11; CI in A549 = 0.19 ±0.11
NA = not achieved
EOHSATD: Excess Over Highest Single Agent at Total Dose, measured as a percentage
EOHSA: Excess over Highest Single Agent, measured as a percentage

**Assay 2**

In Assay 2 the MEK inhibiting compound used is Compound A, as defined herein, and the mTOR inhibiting compound used is Rapamycin, which has the structure indicated below.

Rapamycin:

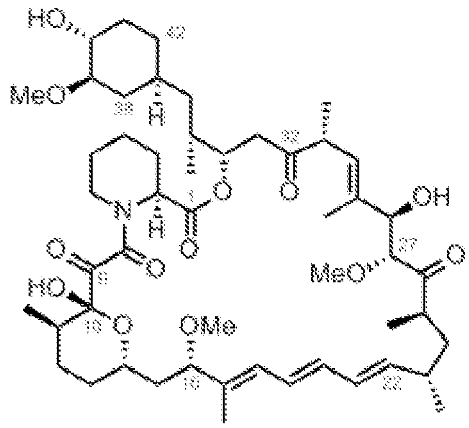

Rapamycin

**Combination of MEK (Compound A) and mTOR (Rapamycin) inhibitors on cancer cells lines.**

95 cancer cell lines were treated with MEK inhibitor (Compound A) or mTOR inhibitor (mTORi, Rapamycin) alone or a combination of both agents at a ratio of 1:2 (MEKi:mTORi) and cell proliferation/death was measured after 3 day drug exposure. Briefly, cells were plated in 384-well plates at 500 cells/well in culture media appropriate for each cell type, supplemented with 10% FBS and 1% penicillin/streptomycin, and incubated overnight at 37°C, 5% $CO_2$. Cells were treated with MEK inhibitor (two fold dilutions ranging from 7.3 uM- 0.014 nM) or with mTOR inhibitor (two fold dilutions ranging from 14.6 uM - 0.112 nM) or in combination with both compounds and incubated for 72 hours. Cell growth was measured using the CellTiterGlo (CTG) reagent (Promega) according to the manufacturer's protocol. Data were analyzed using the XLfit (IDBS Ltd.) curve-fitting tool for Microsoft Excel. Growth IC50 (gIC50) and Ymin values were generated using a four-parameter curve fit algorithm (XLFit algorithm #205).

Results were expressed as a percent of the t=0 and plotted against the compound concentration. The T=0 value is normalized to 100% and represents the number of cells at the time of compound addition. The cellular response was determined for each compound by fitting the concentration response with a 4 or 6 parameter curve fit using XLfit software and determining the concentration that inhibited 50% of the cell growth (gIC50) as well as the lower Y value (Ymin) at any compound concentration. Formula below was used to calculate mutually exclusive combination index (CI). Data were reported as logCI, where the logarithm value of CI was calculated.

$$CI= IC_{50} \text{ of } a+b/ IC_{50} \text{ of } a + IC_{50} \text{ of } b+a/ IC_{50} \text{ of } b$$

Where a=MEKi and b=mTORi

Synergy is defined as CI<1, additivity as CI=1 and antagonism as CI>1. Considering the variability of these proliferation assays we arbitrarily defined logCI>0.1 as antagonism, between -0.1 and 0.1 as no significant effect and <-0.1 as synergy.

Analysis of gIC50 demonstrated that for most cell lines, combination of both inhibitors is beneficial, lowering the gIC50 measured compared to that of each inhibitor alone. Growth IC50 (gIC50) of MEK and mTOR inhibitors alone and in combination against cancer cell lines are graphically represented in Figure 3. For graphic representation, a gIC50>7.3uM for MEKi and >14.6uM for mTORi were graphed as 7.3uM and 14.6 uM, respectively. Combination index (CI) was calculated as described above and its logarithmic values are plotted and represented in Figure 4. These data demonstrated that combination between MEKi and mTORi were synergistic on most cell line tested (74/95 cell lines,

77.9%), while this combination was antagonistic on few cell lines tested (8/95 cell lines, 8.4%).

Figure 5 demonstrates that for some cell line, while the combination of MEK and mTOR inhibitor slightly affect the gIC50, the main difference observed lies in the Ymin (maximum inhibition observed), as define as the lower Y value at any compound concentration. In this example, the Ymin of mTOR inhibitor alone, MEK inhibitor alone and the combination of both drugs are 487%, 257% and -73% respectively. Similar analysis was performed for all cell lines and Ymin results are depicted in Figure 6. These data demonstrate that the combination of both drugs caused a greater percentage of cell undergoing death (below the blue line, cytotoxic, 44.2%) than each drug alone (mTORi= 9.5% and MEKi= 20%).

Similar analysis was performed while grouping cells by mutational status based on the KRAS and PI3K. The data depicted in Table 2 demonstrates that mTORi alone is causing cell death (cytotoxic) only on a small subset of the cancer cell line population tested, independent of the KRAS or PI3K mutational status. However, while the MEKi is causing a considerable number of cell lines to undergo death independent of the KRAS status (>20%), it showed lower activity against PI3K mutant compare to PI3K wild-type (WT) cell lines. The combination of both drugs is active in more cell lines in all mutational subgroups, compared to single agent.

Table 2: Percentage of Cancer Cell Lines Treated with Combination of MEK and mTOR Inhibitors Undergoing Death Based on KRAS or PI3K Mutational Status.

| | % ot cells with cytotoxic respond | | | |
|---|---|---|---|---|
| | KRAS WT | KRAS mutant | PI3KWT | PI3K mutant |
| mTORI alone | 10 | 8 | 8 | 12 |
| MEKI alone | 23 | 21 | 29 | 4 |
| mTORi + MEKi | 45 | 46 | 51 | 25 |

Analysis of the capability of each compound alone or in combination to cause cell death based on the KRAS and PI3K mutational status simultaneously is depicted in Table 3. While mTORi as a single agent caused few cell lines to undergo death (≤11%), the MEKi alone induced cell death in 30% of cell lines with KRAS mutant/PI3K WT genotype. The combination of both agents, in addition to being superior in the total number of cell lines undergoing death, is active against not only KRAS mutant/PI3K WT population but on cells with wild type KRAS as well as cells encoding a mutant PI3K.

Table 3: Percentage of Cancer Cell Lines Treated with Combination of MEK and mTOR Inhibitors Undergoing Death Based on KRAS and PI3K Mutational Status.

| | | %of cells with cytotoxic response | |
|---|---|---|---|
| | | PI3K WT | PI3K mutant |
| mTORi | KRAS WT | 10 | 5 |
| | KRAS mutant | 11 | 6 |
| MEKi | KRAS WT | 7 | 6 |
| | KRAS mutant | **30** | 0 |
| combo | KRAS WT | **50** | **33** |
| | KRAS mutant | **46** | 0 |

In summary, the combination of MEKi and mTORi is advantageous in reducing the concentration of each compound to cause 50% growth inhibition, resulting in a synergistic effect on 77.9% of cancer cell lines tested. Moreover, this combination caused cell death, as measure by the Ymin value compared to initial cell number at T=0, in more cancer cell lines than each agent alone. The combination of these agents was shown to be advantageous in KRAS WT cancer cell lines independent of PI3K mutational status and on KRAS mutant cell lines with wild type PI3K.

Because the combinations of the present invention are active in the above assays they exhibit advantageous therapeutic utility in treating cancer.

Suitably, the present invention relates to a method for treating or lessening the severity of a cancer selected from: brain (gliomas), glioblastomas, astrocytomas, glioblastoma multiforme, Bannayan-Zonana syndrome, Cowden disease, Lhermitte-Duclos disease, breast, inflammatory breast cancer, Wilm's tumor, Ewing's sarcoma, Rhabdomyosarcoma, ependymoma, medulloblastoma, colon, head and neck, kidney, lung, liver, melanoma, ovarian, pancreatic,

prostate, sarcoma, osteosarcoma, giant cell tumor of bone, thyroid,

Lymphoblastic T cell leukemia, Chronic myelogenous leukemia, Chronic lymphocytic leukemia, Hairy-cell leukemia, acute lymphoblastic leukemia, acute myelogenous leukemia, Chronic neutrophilic leukemia, Acute lymphoblastic T cell leukemia, Plasmacytoma, Immunoblastic large cell leukemia, Mantle cell leukemia, Multiple myeloma Megakaryoblastic leukemia, multiple myeloma, acute megakaryocytic leukemia, promyelocytic leukemia, Erythroleukemia,

malignant lymphoma, hodgkins lymphoma, non-hodgkins lymphoma, lymphoblastic T cell lymphoma, Burkitt's lymphoma, follicular lymphoma,

neuroblastoma, bladder cancer, urothelial cancer, lung cancer, vulval cancer, cervical cancer, endometrial cancer, renal cancer, mesothelioma, esophageal cancer, salivary gland cancer, hepatocellular cancer, gastric cancer, nasopharangeal cancer, buccal cancer, cancer of the mouth, GIST (gastrointestinal stromal tumor) and testicular cancer.

Suitably, the present invention relates to a method for treating or lessening the severity of a cancer selected from: brain (gliomas), glioblastomas, astrocytomas, glioblastoma multiforme, Bannayan-Zonana syndrome, Cowden disease, Lhermitte-Duclos disease, breast, colon, head and neck, kidney, lung, liver, melanoma, ovarian, pancreatic, prostate, sarcoma and thyroid.

Suitably, the present invention relates to a method for treating or lessening the severity of a cancer selected from ovarian, breast, pancreatic and prostate.

Suitably, the present invention relates to a method of treating or lessening the severity of a cancer that is either wild type or mutant for Ras/Raf and either wild type or mutant for PI3K/Pten. This includes patients wild type for both Ras/Raf and PI3K/PTEN, mutant for both Ras/Raf and PI3K1PTEN, mutant for Ras/Raf and wild type for PI3K1PTEN and wild type for Ras/Raf and mutant for PI3K/PTEN.

In one embodiment, the tumor cell also has at least one Braf mutation. Braf mutation include: R462I, I463S, G464V, G464E, G466A, G466E, G466V, G469A, G469E, D594V, F595L, G596R, L597V, L597R, T599I, V600E, V600D, V600K, V600R, T119S, and K601 E.

The term "wild type" as is understood in the art refers to a polypeptide or polynucleotide sequence that occurs in a native population without genetic modification. As is also understood in the art, a "mutant" includes a polypeptide or polynucleotide sequence having at least one modification to an amino acid or nucleic acid compared to the corresponding amino acid or nucleic acid found in a wild type polypeptide or polynucleotide, respectively. Included in the term mutant is Single Nucleotide Polymorphism (SNP) where a single base pair distinction exists in the sequence of a nucleic acid strand compared to the most prevalently found (wild type) nucleic acid strand.

Cancers that are either wild type or mutant for Ras/Raf and either wild type or mutant for PI3K/Pten are identified by known methods.

For example, wild type or mutant Ras/Raf or PI3K/PTEN tumor cells can be identified by DNA amplification and sequencing techniques, DNA and RNA detection techniques, including, but not limited to Northern and Southern blot, respectively, and/or various biochip and array technologies. Wild type and mutant polypeptides can be detected by a variety of techniques including, but not limited to immunodiagnostic techniques such as ELISA, Western blot or imunocyto chemistry.

Germline mutation in serine/threonine kinase 11 (STK11, also called LKB1) results in Peutz-Jeghers syndrome, characterized by intestinal hamartomas and increased incidence of epithelial cancers. (Hongbin, et al. Nature (2007) 448:807-810 and Hearle, et al,. Clin. Cancer Res. (2006) 12:3209-3215). Somatic LKB1 mutations have been reported in some primary human lung adenocarcinomas and has been shown to modulate cell differentiation and metastasis in Kras mutated lung cancer (Hongbin, et *al.*)

As used herein "LKB1" is synonymous with Serine/Threonine Kinase 11 (STK11). The human LKB gene (official HUGO symbol, STK11) encodes a serine/threonine protein kinase that is defective in patients with Peutz-Jeghers syndrome (PJS). PJS is an autosomal dominantly inherited syndrome characterized by hamartomatous polyposis of the gastrointestinal tract and mucocutaneous pigmentation. To date, 145 different germline LKB1 mutations have been reported. The majority of the mutations lead to a truncated protein product. One mutational hotspot has been observed. A 1-bp deletion and a 1-bp insertion at the mononucleotide repeat (C6 repeat, c.837-c.842) between the codons 279-281 have been found in six and seven unrelated PJS families, respectively. However, these mutations account only for approximately 7% of all mutations identified in the PJS families (13/193). A review of the literature provides a total of 40 different somatic LKB1 mutations in 41 sporadic tumors and seven cancer cell lines. Mutations occur particularly in lung and colorectal cancer. Most of the somatic LKB1 mutations result in truncation of the protein. A mutational hotspot seems to be a C6 repeat accounting for 12.5% of all somatic mutations (6/48). These results are concordant with the germline mutation spectrum. However, the proportion of the missense mutations seems to be higher among the somatic mutations (45%) than among the germline mutations (21%), and only seven of the mutations are exactly the same in both of the mutation types. Hum Mutat 26(4), 291-297, 2005. Launonen. Human Mutation. 26(4), 291-297, 2005.

The term "Ras protein" as used herein means any protein which is a member of the ras subfamily which is a subfamily of GTPases involved in cellular signaling. As is known in the art, activation of Ras causes cell growth, differentiation and survival. Ras proteins include, but are not limited to, H-ras, K-ras and N-ras.

In one aspect, tumor cells have at least one mutation in at least one Ras protein or gene encoding at least one Ras protein is in K-ras, N-ras or H-ras. A Ras mutation in at least one gene encoding at least one Ras protein may be in exon 2 and/or 3. In some instances, a gene encoding at least one Ras protein has a mutation in at least one of ras codon selected from: codon 12, 13, 14, 60, 74 and 76. In some embodiments, a Ras mutation is selected from: G12S, G12V, G12D, G12A, G12C, G12R, G13A, G13D, V14I, G60E, T74P, E76G, E76K and E76Q.

Tumor cells may have a mutation, deletion or insertion in LKB1. At least one missense mutation in LKB1 may be selected from: 581A>T causing amino acid change D194V; 842C>T causing amino acid change P281L; 595G>C causing amino acid change E199Q; 1062C>G causing amino acid change F354L; 521A>G causing amino acid change H174R; 526G>T causing amino acid change D176Y; 580G>T causing amino acid change D194Y; 580G>A causing amino acid change D194N; 166G>T causing amino acid change G56W;167G>T causing amino acid change G56V; 587G>T causing amino acid change G196Y; 232A>G causing amino acid change K78E; 724G>C causing amino acid change G242R; 725G>T causing amino acid change G242V; 709G>T causing amino acid change D237Y; 910C>G causing amino acid change R304G; 829G>T causing amino acid change D277Y; 923G>T causing amino acid change W308L; 854T>A causing amino acid change L285Q; 1225C>T causing amino acid change R409W; 256C>G causing amino acid change R86G; 1062C>G causing amino acid change F354L; 816C>T causing amino acid change Y272Y; 487G>T causing amino acid change G163C; 368A>G causing amino acid change Q123R and/or 1276C>T causing amino acid change R426W.

In another embodiment, at least one nonsense mutation in LKB1 is selected from: 109C>T causing amino acid change Q37X; 508C>T causing amino acid change Q170X; 206C>A causing amino acid change S69X; 358G>T causing amino acid change E120X; 180C>G causing amino acid change Y60X; 180C>A causing amino acid change Y60X; 595G>T causing amino acid change E199X; 409C>T causing amino acid change Q137X; 493G>T causing amino acid change E165X; 571A>T causing amino acid change K191X; 658C>T causing amino acid change Q220X; 193G>T causing amino acid change E65X; 130A>T causing amino acid change K44X; 630C>A causing amino acid change C210X; 667G>T causing amino acid change E223X; 208G>T causing amino acid change E70X; 996G>A causing amino acid change W332X; 949G>T causing amino acid change E317X; 996G>A causing amino acid change W332X; 658C>T causing amino acid change Q220X and/or 475C>T causing amino acid change Q159X.

In another embodiment, at least one deletion, insertion, substitution or complex mutation in LKB1 is selected from: 120_130del11; 153delG; 126_149del24; 291_464del174; 291_597del307; 465_597del133; 842delC; 735_862del128; 166_178del13; 431delC; 579delC; 157delG; 810delG; 598_13del22; 544_546delCTG; 827delG; 169delG; 291_378del88; 598delG; 842delC; 465_862del1398; 633delG; 1302del1302; 379_433del55; 128_129delC; 142_143delA; 180delC; 209delA; 227_228delC; 47_651del605; 153_536del384; exon 2-3del; exon 2-3del; exon 2-3del; exon 2-4del; 562_563delG; exon 4del; exon 4del; exon 4del; exon 4del; 610_623del14; 837delC; 464_465del2GGinsTTTGCT; 75-76del2&insT; 125_127insGG; 584_585insT; 704_705insA; 152_153insCT; 842_843insC; 649_650insG; 127 128insGG; 979_980insAG; 165_166insT; exon 6del; and/or 1039_1040ins; 735-2A>T; 5982AT; 465-1G>A; 465-1G>T; 291-2A>T; 921-1G>A; and/or 597+1G>T; 143_144>T; 841842>T; and/or 271272GG>TT.

In another embodiment, the deletion, insertion or mutation of LKB1 is in the catalytic kinase domain. The deletion, insertion or mutation of LKB1 may be in codons 50-337.

This invention provides a combination comprising N-{3-[3-cyclopropyl-5-(2-fluoro-4-iodo-phenylamino)6,8-dimethyl-2,4,7-trioxo-3,4,6,7-tetrahydro-2H-pyrido[4,3-d]pyrimidin-1-yl]phenyl}acetamide, or a pharmaceutically acceptable salt or solvate thereof, and an mTOR inhibiting compound, suitably everolimus.

This invention also provides for a combination comprising N-{3-[3-cyclopropyl-5-(2-fluoro-4-iodo-phenylamino)6,8-dimethyl-2,4,7-trioxo-3,4,6,7-tetrahydro-2H-pyrido[4,3-d]pyrimidin-1-yl]phenyl}acetamide, or a pharmaceutically acceptable salt or solvate thereof, and an mTOR inhibiting compound, suitably everolimus, for use in therapy.

This invention also provides for a combination comprising N-{3-[3-cyclopropyl-5-(2-fluoro-4-iodo-phenylamino)6,8-dimethyl-2,4,7-trioxo-3,4,6,7-tetrahydro-2H-pyrido[4,3-d]pyrimidin-1-yl]phenyl}acetamide, or a pharmaceutically acceptable salt or solvate thereof, and an mTOR inhibiting compound, suitably everolimus, for use in treating cancer.

This invention also provides a pharmaceutical composition comprising a combination of N-{3-[3-cyclopropyl-5-(2-fluoro-4-iodo-phenylamino)6,8-dimethyl-2,4,7-trioxo-3,4,6,7-tetrahydro-2H-pyrido[4,3-d]pyrimidin-1-yl]phenyl}acetamide, or a pharmaceutically acceptable salt or solvate thereof, and an mTOR inhibiting compound, suitably everolimus.

This invention also provides a combination kit comprising N-{3-[3-cyclopropyl-5-(2-fluoro-4-iodo-phenylamino)6,8-dimethyl-2,4,7-trioxo-3,4,6,7-tetrahydro-2H-pyrido[4,3-d]pyrimidin-1-yl]phenyl}acetamide, or a pharmaceutically acceptable salt or solvate thereof, and an mTOR inhibiting compound, suitably everolimus.

This invention also provides for the use of a combination comprising N-{3-[3-cyclopropyl-5-(2-fluoro-4-iodo-phenylamino)6,8-dimethyl-2,4,7-trioxo-3,4,6,7-tetrahydro-2H-pyrido[4,3-d]pyrimidin-1-yl]phenyl}acetamide, or a pharmaceutically acceptable salt or solvate thereof, and an mTOR inhibiting compound, suitably everolimus, in the manufacture of a medicament.

This invention also provides for the use of a combination comprising N-{3-[3-cyclopropyl-5-(2-fluoro-4-iodo-phenylamino)6,8-dimethyl-2,4,7-trioxo-3,4,6,7-tetrahydro-2H-pyrido[4,3-d]pyrimidin-1-yl]phenyl}acetamide, or a pharmaceutically acceptable salt or solvate thereof, and an mTOR inhibiting compound, suitably everolimus, in the manufacture of a medicament to treat cancer.

This invention also provides a method of treating cancer which comprises administering a combination of N-{3-[3-cyclopropyl-5-(2-fluoro-4-iodo-phenylamino)6,8-dimethyl-2,4,7-trioxo-3,4,6,7-tetrahydro-2H-pyrido[4,3-d]pyrimidin-1-yl]phenyl}acetamide, or a pharmaceutically acceptable salt or solvate thereof, and an mTOR inhibiting compound, suitably everolimus, to a subject in need thereof.

The following examples are intended for illustration only and are not intended to limit the scope of the invention in any way.

## EXAMPLES

### Example 1 - Capsule Composition

An oral dosage form for administering a combination of the present invention is produced by filing a standard two piece hard gelatin capsule with the ingredients in the proportions shown in Table 4, below.

Table 4

| INGREDIENTS | AMOUNTS |
|---|---|
| N-{3-[3-cyclopropyl-5-(2-fluoro-4-iodo-phenylamino)-6,8-dimethy;-2,4,7-trioxo-3,4,6,7-tetrahydro-2H-pyrido[4,3-d]pyrimidin-1-yl]phenyl}acetamide dimethyl sulfoxide (the dimethyl sulfoxide solvate of Compound A) | 5mg |
| everolimus | 5mg |
| Mannitol | 250 mg |
| Talc | 125 mg |
| Magnesium Stearate | 8mg |

### Example 2 - Capsule Composition

An oral dosage form for administering one of the compounds of the present invention is produced by filing a standard two piece hard gelatin capsule with the ingredients in the proportions shown in Table 5, below.

Table 5

| INGREDIENTS | AMOUNTS |
|---|---|
| N-{3-[3-cyclopropyl-5-(2-fluoro-4-iodo-phenylamino)-6,8-dimethy;-2,4,7-trioxo-3,4,6,7-tetrahydro-2H-pyrido[4,3-d]pyrimidin-1-yl]phenyl}acetamide dimethyl sulfoxide (the dimethyl sulfoxide solvate of Compound A) | 5mg |
| Mannitol | 55 mg |
| Talc | 16 mg |
| Magnesium Stearate | 4 mg |

### Example 3 - Capsule Composition

An oral dosage form for administering one of the compounds of the present invention is produced by filing a standard two piece hard gelatin capsule with the ingredients in the proportions shown in Table 6, below.

Table 6

| INGREDIENTS | AMOUNTS |
|---|---|
| everolimus | 5mg |
| Mannitol | 250mg |
| Talc | 125mg |
| Magnesium Stearate | 8mg |

Example 4 - Tablet Composition

The sucrose, microcrystalline cellulose and the compounds of the invented combination, as shown in Table 7 below, are mixed and granulated in the proportions shown with a 10% gelatin solution. The wet granules are screened, dried, mixed with the starch, talc and stearic acid, then screened and compressed into a tablet.

Table 7

| INGREDIENTS | AMOUNTS |
|---|---|
| N-{3-[3-cyclopropyl-5-(2-fluoro-4-iodo-phenylamino)-6,8-dimethy;-2,4,7-trioxo-3,4,6,7-tetrahydro-2H-pyrido[4,3-d]pyrimidin-1-yl]phenyl}acetamide dimethyl sulfoxide (the dimethyl sulfoxide solvate of Compound A) | 5mg |
| everolimus | 5mg |
| Microcrystalline cellulose | 300mg |
| Sucrose | 10mg |
| Starch | 40mg |
| Talc | 20mg |
| stearic acid | 5mg |

Example 5 - Tablet Composition

The sucrose, microcrystalline cellulose and one of the compounds of the invented combination, as shown in Table 8 below, are mixed and granulated in the proportions shown with a 10% gelatin solution. The wet granules are screened, dried, mixed with the starch, talc and stearic acid, then screened and compressed into a tablet.

Table 8

| INGREDIENTS | AMOUNTS |
|---|---|
| N-{3-[3-cyclopropyl-5-(2-fluoro-4-iodo-phenylamino)-6,8-dimethy;-2,4,7-trioxo-3,4,6,7-tetrahydro-2H-pyrido[4,3-d]pyrimidin-1-yl]phenyl}acetamide dimethyl sulfoxide (the dimethyl sulfoxide solvate of Compound A) | 5mg |
| Microcrystalline cellulose | 30mg |
| sucrose | 4mg |
| starch | 2mg |
| talc | 1mg |
| stearic acid | 0.5mg |

Example 6 - Tablet Composition

The sucrose, microcrystalline cellulose and one of the compounds of the invented combination, as shown in Table 9 below, are mixed and granulated in the proportions shown with a 10% gelatin solution. The wet granules are screened, dried, mixed with the starch, talc and stearic acid, then screened and compressed into a tablet.

Table 9

| INGREDIENTS | AMOUNTS |
|---|---|
| everolimus | 5mg |
| Microcrystalline cellulose | 300mg |
| sucrose | 40mg |
| starch | 20mg |
| talc | 10mg |
| stearic acid | 5mg |

While the preferred embodiments of the invention are illustrated by the above, it is to be understood that the invention is not limited to the precise instructions herein disclosed and that the right to all modifications coming within the scope of the following claims is reserved.

## Claims

1. A combination comprising:

    (i) N-{3-[3-cyclopropyl-5-(2-fluoro-4-iodo-phenylamino)6,8-dimethyl;-2,4,7-trioxo-3,4,6,7-tetrahydro-2H-pyrido[4,3-d]pyrimidin-1-yl]phenyl}acetamide (a compound of structure (I)):

    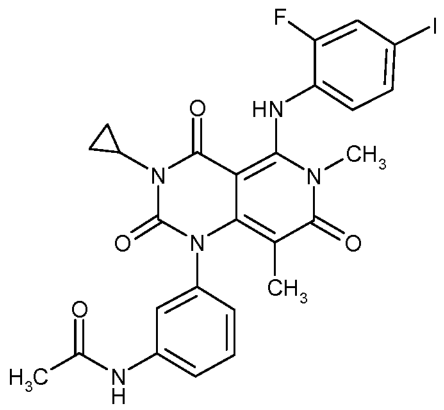

    (I);

    or a pharmaceutically acceptable salt or solvate thereof; and
    (ii) everolimus.

2. A combination according to claim 1 where the compound of Structure (I) is in the form of a dimethyl sulfoxide solvate.

3. A pharmaceutical composition comprising a combination according to claim 1 or claim 2 together with a pharmaceutically acceptable diluent or carrier.

4. A combination kit comprising a combination according to claim 1 or claim 2 together with a pharmaceutically acceptable carrier or carriers.

5. A combination as claimed in claim 1 or claim 2 for use in therapy.

6. A combination according to claim 1 or claim 2 for use in treating cancer.

7. A combination according to claim 1 or claim 2 for use in treating cancer, wherein the combination is administered within a specified period and wherein the combination is administered for a duration of time.

8. A combination for use according to claim 7 wherein the amount of N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide is selected from about 0.25mg to about 9mg, and that amount is administered once per day, and the amount of everolimus is selected from about 3mg to about 15mg, and that amount is administered once per day.

9. A combination for use according to claim 8 wherein N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide and everolimus are administered within 12 hours of each other for from 1 to 3 consecutive days followed by administration of N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide for from 3 to 7 consecutive days, optionally followed by one or more cycles of repeat dosing.

10. A combination for use according to claim 7 wherein N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide and everolimus are administered within 12 hours of each other for from 1 to 3 consecutive days followed by administration of everolimus for from 4 to 6 consecutive days, optionally followed by one or more cycles of repeat dosing.

11. A combination for use according to claim 7 wherein N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide and everolimus are administered within 12 hours of each other for 2 days over a 7 day period, and during the other days of the 7 day period: either N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide; or everolimus, is administered alone, optionally followed by one or more cycles of repeat dosing.

**12.** A combination for use according to claim 7 wherein N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide and everolimus are administered within 12 hours of each other for at least 5 consecutive days.

**13.** A combination for use according to claim 7 wherein N-{3-[3-cyclopropyl-5-(2-fluoro-4-iodo-phenylamino)-6,8-dimethyl-2,4,7-trioxo-3,4,6,7-tetrahydro-2H- pyrido[4,3-d]pyrimidin-1-yl]phenyl}acetamide dimethyl sulfoxide and everolimus are administered within 12 hours of each other for 5 days over a 14 day period, and during the other days of the 14 day period: either N-{3-[3-cyclopropyl-5-(2-fluoro-4-iodo-phenylamino)-6,8-dimethyl-2,4,7-trioxo-3,4,6,7-tetrahydro-2H-pyrido[4,3- d]pyrimidin-1-yl]phenyl}acetamidedimethyl sulfoxide is administeredalone, optionally followed by one or more cycles or repeat dosing; or everolimus, is administered alone, optionally followed by one or more cycles of repeat dosing.

**14.** A combination for use according to claim 7 wherein the compound N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide is first administered in a loading dose for from 1 to 3 days followed by maintenance dose administration of the compound, and/or the compound everolimus is first administered in a loading dose for from 1 to 3 days followed by maintenance dose administration of the compound.

**15.** A combination for use according to any one of claims 5 to 14, wherein the cancer is either wild type or mutant for Ras/Raf and either wild type or mutant for PI3K/PTEN.

**16.** A combination for use according to any one of claims 5 to 14, wherein the cancer is selected from: brain (gliomas), glioblastomas, astrocytomas, glioblastoma multiforme, Bannayan-Zonana syndrome, Cowden disease, Lhermitte-Duclos disease, breast, inflammatory breast cancer, Wilm's tumor, Ewing's sarcoma, Rhabdomyosarcoma, ependymoma, medulloblastoma, colon, head and neck, kidney, lung, liver, melanoma, ovarian, pancreatic, prostate, sarcoma, osteosarcoma, giant cell tumor of bone, thyroid, Lymphoblastic T cell leukemia, Chronic myelogenous leukemia, Chronic lymphocytic leukemia, Hairy-cell leukemia, acute lymphoblastic leukemia, acute myelogenous leukemia, Chronic neutrophilic leukemia, Acute lymphoblastic T cell leukemia, Plasmacytoma, Immunoblastic large cell leukemia, Mantle cell leukemia, Multiple myeloma Megakaryoblastic leukemia, multiple myeloma, acute megakaryocytic leukemia, promyelocytic leukemia, Erythroleukemia, malignant lymphoma, hodgkins lymphoma, non-hodgkins lymphoma, lymphoblastic T cell lymphoma, Burkitt's lymphoma, follicular lymphoma, neuroblastoma, bladder cancer, urothelial cancer, lung cancer, vulval cancer, cervical cancer, endometrial cancer, renal cancer, mesothelioma, esophageal cancer, salivary gland cancer, hepatocellular cancer, gastric cancer, nasopharangeal cancer, buccal cancer, cancer of the mouth, GIST (gastrointestinal stromal tumor) and testicular cancer.

## Patentansprüche

**1.** Kombination umfassend:

(i) N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamid (eine Verbindung der Struktur (I)):

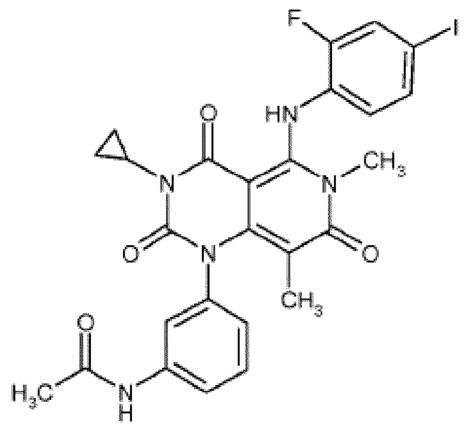

(I);

oder ein pharmazeutisch akzeptables Salz oder Solvat davon; und
(ii) Everolimus.

**2.** Kombination nach Anspruch 1, wobei die Verbindung der Struktur (I) in Form eines Dimethylsulfoxid-Solvats ist.

**3.** Pharmazeutische Zusammensetzung umfassend eine Kombination nach Anspruch 1 oder Anspruch 2 zusammen mit einem pharmazeutisch akzeptablen Verdünnungsmittel oder Träger.

**4.** Kombinationskit umfassend eine Kombination nach Anspruch 1 oder Anspruch 2 zusammen mit einem pharmazeutisch akzeptablen Träger oder mit pharmazeutisch akzeptablen Trägern.

**5.** Kombination nach Anspruch 1 oder Anspruch 2 für deren Verwendung in der Therapie.

**6.** Kombination nach Anspruch 1 oder Anspruch 2 für deren Verwendung bei der Behandlung von Krebs.

**7.** Kombination nach Anspruch 1 oder Anspruch 2 für deren Verwendung bei der Behandlung von Krebs, wobei die Kombination innerhalb eines festgelegten Zeitraums verabreicht wird und wobei die Kombination während einer Zeitdauer verabreicht wird.

**8.** Kombination für deren Verwendung nach Anspruch 7, wobei die Menge von N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamid-Dimethylsulfoxid aus etwa 0,25 mg bis etwa 9 mg ausgewählt wird, und diese Menge einmal pro Tag verabreicht wird, und die Menge von Everolimus aus etwa 3 mg bis etwa 15 mg ausgewählt wird, und diese Menge einmal pro Tag verabreicht wird.

**9.** Kombination für deren Verwendung nach Anspruch 8, wobei N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamid-Dimethylsulfoxid und Everolimus im Abstand von 12 Stunden voneinander für 1 bis 3 aufeinanderfolgende Tage verabreicht werden, gefolgt von einer Verabreichung von N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamid-Dimethylsulfoxid für 3 bis 7 aufeinanderfolgende Tage, gegebenenfalls gefolgt von einem oder mehreren Zyklen wiederholter Dosierung.

**10.** Kombination für deren Verwendung nach Anspruch 7, wobei N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamid-Dimethylsulfoxid und Everolimus im Abstand von 12 Stunden voneinander für 1 bis 3 aufeinanderfolgende Tage verabreicht werden, gefolgt von einer Verabreichung von Everolimus für 4 bis 6 aufeinanderfolgende Tage, gegebenenfalls gefolgt von einem oder mehreren Zyklen wiederholter Dosierung.

**11.** Kombination für deren Verwendung nach Anspruch 7, wobei N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamid-Dimethylsulfoxid und Everolimus im Abstand von 12 Stunden voneinander für 2 Tage über einen Zeitraum von 7 Tagen verabreicht werden, und während der anderen Tage des Zeitraums von 7 Tagen: entweder N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamid-Dimethylsulfoxid; oder Everolimus, allein verabreicht wird, gegebenenfalls gefolgt von einem oder mehreren Zyklen wiederholter Dosierung.

**12.** Kombination für deren Verwendung nach Anspruch 7, wobei N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamid-Dimethylsulfoxid und Everolimus im Abstand von 12 Stunden voneinander für mindestens 5 aufeinanderfolgende Tage verabreicht werden.

**13.** Kombination für deren Verwendung nach Anspruch 7, wobei N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamid-Dimethylsulfoxid und Everolimus im Abstand von 12 Stunden voneinander für 5 Tage über einen Zeitraum von 14 Tagen verabreicht werden, und während der anderen Tage des Zeitraums von 14 Tagen: entweder N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamid-Dimethylsulfoxid allein verabreicht wird, gegebenenfalls gefolgt von einem oder mehreren Zyklen wiederholter Dosierung; oder Everolimus allein verabreicht wird, gegebenenfalls gefolgt von einem oder mehreren Zyklen wiederholter Dosierung.

**14.** Kombination für deren Verwendung nach Anspruch 7, wobei die Verbindung N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamid-Dimethylsulfoxid zuerst in einer Anfangsdosis für 1 bis 3 Tage verabreicht wird, gefolgt von der Verabreichung einer Erhaltungsdosis der Verbindung, und/oder die Verbindung Everolimus zuerst in einer Anfangsdosis für 1 bis 3 Tage verabreicht wird, gefolgt von der Verabreichung einer Erhaltungsdosis der Verbindung.

**15.** Kombination für deren Verwendung nach einem der Ansprüche 5 bis 14, wobei der Krebs entweder vom Wildtyp oder eine Mutation für Ras/Raf und entweder vom Wildtyp oder eine Mutation für PI3K/PTEN ist.

**16.** Kombination für deren Verwendung nach einem der Ansprüche 5 bis 14, wobei der Krebs aus Folgendem ausgewählt wird: Hirntumoren (Gliomen), Glioblastomen, Astrozytomen, Glioblastoma multiforme, Bannayan-Zonana-Syndrom, Cowden-Krankheit, Lhermitte-Duclos-Krankheit, Brustkrebs, entzündlichem Brustkrebs, Wilms-Tumor, Ewing-Sarkom, Rhabdomyosarkom, Ependymom, Medulloblastom, Dickdarm-, Kopf-Hals-, Nieren-, Lungen-, Leberkrebs, Melanom, Eierstock-, Bauchspeicheldrüsen-, Prostatakrebs, Sarkom, Osteosarkom, Riesenzellentumor in Knochen, Schilddrüsenkrebs, lymphoblastischer T-Zell-Leukämie, chronischer myeloischer Leukämie, chronischer lymphatischer Leukämie, Haarzellleukämie, akuter lymphatischer Leukämie, akuter myeloischer Leukämie, chronischer Neutrophilenleukämie, akuter lymphoblastischer T-Zell-Leukämie, Plasmozytom, immunoblastischer Großzellen-Leukämie, Mantelzell-Leukämie, multiplem Myelom, megakaryoblastischer Leukämie, multiplem Myelom, akuter megakaryozytischer Leukämie, promyelozytischer Leukämie, Erythroleukämie, malignem Lymphom, Hodgkin-Lymphom, Non-Hodgkin-Lymphom, lymphoblastischem T-Zell-Lymphom, Burkitt-Lymphom, follikulärem Lymphom, Neuroblastom, Blasenkrebs, urothelialem Krebs, Lungenkrebs, Vulvakrebs, Zervixkrebs, Gebärmutterkrebs, Nierenkrebs, Mesotheliom, Speiseröhrenkrebs, Speicheldrüsenkrebs, hepatozellulärem Krebs, Magenkrebs, Nasenrachenkrebs, bukkalem Krebs, Mundkrebs, GIST (gastrointestinalem Stromatumor) und Hodenkrebs.

**Revendications**

**1.** Une combinaison comprenant:

(i) N-{3-[3-cyclopropyl-5-(2-fluoro-4-iodo-phénylamino)6,8-diméthyl-2,4,7-trioxo-3,4,6,7-tétrahydro-2H-pyrido[4,3-d]pyrimidin-1-yl]phényl}acétamide (un composé de structure (I)):

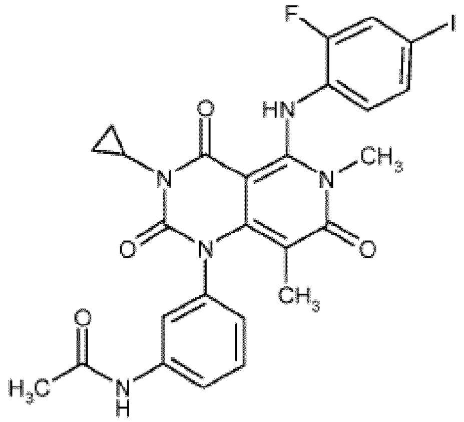

(I);

ou un de ses sels ou solvates pharmaceutiquement acceptables; et
(ii) évérolimus.

2. Une combinaison selon la revendication 1, dans laquelle le composé de structure (I) est sous la forme d'un solvate de diméthylsulfoxyde.

3. Une composition pharmaceutique comprenant une combinaison selon la revendication 1 ou la revendication 2 conjointement avec un diluant ou un support pharmaceutiquement acceptable.

4. Une combinaison comprenant une combinaison selon la revendication 1 ou la revendication 2 conjointement avec un ou plusieurs supports pharmaceutiquement acceptables.

5. Une combinaison selon la revendication 1 ou la revendication 2 pour une utilisation en thérapie.

6. Une combinaison selon la revendication 1 ou la revendication 2 pour une utilisation dans le traitement du cancer.

7. Une combinaison selon la revendication 1 ou la revendication 2 pour une utilisation dans le traitement du cancer, dans laquelle la combinaison est administré dans une période spécifiée et dans laquelle la combinaison est administrée pendant une durée de temps.

8. Une combinaison pour une utilisation selon la revendication 6, dans laquelle la quantité de diméthylsulfoxyde N-{3-[3-cyclopropyl-5-(2-fluoro-4-iodo-phénylamino)-6,8-diméthyl-2,4,7-trioxo-3,4,6,7-tétrahydro-2H-pyrido[4,3-d]pyrimidin-1-yl]phényl}acétamide est choisi entre environ 0,25 mg et environ 9 mg, et cette quantité est administrée une fois par jour, et la quantité d'évérolimus est choisie entre environ 3 mg et environ 15 mg et cette quantité est administrée une fois par jour.

9. Une combinaison pour une utilisation selon la revendication 8, dans laquelle le diméthylsulfoxyde N-{3-[3-cyclopropyl-5-(2-fluoro-4-iodo-phénylamino)-6,8-diméthyl-2,4,7-trioxo-3,4,6,7-tétrahydro-2H-pyrido[4,3-d]pyrimidin-1-yl]phényl}acetamide et l'évérolimus sont administrés dans une intervalle de temps de 12 heures l'un par rapport à l'autre pendant 1 à 3 jours consécutifs suivi de l'administration de diméthylsulfoxyde N-{3-[3-cyclopropyl-5-(2-fluoro-4-iodo-phénylamino)-6,8-diméthyl-2,4,7-trioxo-3,4,6,7-tétrahydro-2H-pyrido[4,3-d]pyrimidin-1-yl]phényl}acétamide pendant 3 à 7 jours consécutifs, optionnellement suivi d'un ou plusieurs cycles de dosage répété.

10. Une combinaison pour une utilisation selon la revendication 7, dans laquelle le diméthylsulfoxyde N-{3-[3-cyclopropyl-5-(2-fluoro-4-iodo-phénylamino)-6,8-diméthyl-2,4,7-trioxo-3,4,6,7-tétrahydro-2H-pyrido[4,3-d]pyrimidin-1-yl]phényl}acétamide et l'évérolimus sont administrés dans une intervalle de temps de 12 heures l'un par rapport à l'autre pendant 1 à 3 jours consécutifs suivi de l'administration d'évérolimus pendant 4 à 6 jours consécutifs, optionnellement suivi d'un ou plusieurs cycles de dosage répété.

11. Une combinaison pour une utilisation selon la revendication 7, dans laquelle le diméthylsulfoxyde N-{3-[3-cyclopropyl-5-(2-fluoro-4-iodo-phénylamino)-6,8-diméthyl-2,4,7-trioxo-3,4,6,7-tétrahydro-2H-pyrido[4,3-d]pyrimidin-1-yl]phényl}acétamide et l'évérolimus sont administrés dans une intervalle de temps de 12 heures l'un par rapport à l'autre pendant 2 jours au cours d'une période de 7 jours et pendant les autres jours de la période de 7 jours: soit

le diméthylsulfoxyde N-{3-[3-cyclopropyl-5-(2-fluoro-4-iodo-phénylamino)-6,8-diméthyl-2,4,7-trioxo-3,4,6,7-tétrahydro-2H-pyrido[4,3-d]pyrimidin-1-yl]phényl}acetamide; soit l'évérolimus, est administré seul, optionnellement suivi d'un ou plusieurs cycles de dosage répété.

**12.** Une combinaison pour une utilisation selon la revendication 7, dans laquelle le diméthylsulfoxyde N-{3-[3-cyclopropyl-5-(2-fluoro-4-iodo-phénylamino)-6,8-diméthyl-2,4,7-trioxo-3,4,6,7-tétrahydro-2H-pyrido[4,3-d]pyrimidin-1-yl]phényl}acétamide et l'évérolimus sont administrés dans une intervalle de temps de 12 heures l'un par rapport à l'autre pendant au moins 5 jours consécutifs.

**13.** Une combinaison pour une utilisation selon la revendication 7, dans laquelle le diméthylsulfoxyde N-{3-[3-cyclopropyl-5-(2-fluoro-4-iodo-phénylamino)-6,8-diméthyl-2,4,7-trioxo-3,4,6,7-tétrahydro-2H-pyrido[4,3-d]pyrimidin-1-yl]phenyl}acétamide et l'évérolimus sont administrés dans une intervalle de temps de 12 heures l'un par rapport à l'autre pendant 5 jours au cours d'une période de 14 jours, et pendant les autres jours de la période de 14 jours: soit le diméthylsulfoxyde N-{3-[3-cyclopropyl-5-(2-fluoro-4-iodo-phénylamino)-6,8-diméthyl-2,4,7-trioxo-3,4,6,7-tétrahydro-2H-pyrido[4,3-d]pyrimidin-1-yl]phényl}acétamide est administré seul, optionnellement suivi d'un ou plusieurs cycles ou un dosage répété; soit l'évérolimus, est administré seul, optionnellement suivi d'un ou plusieurs cycles de dosage répété.

**14.** Une combinaison pour une utilisation selon la revendication 7, dans laquelle le composé diméthylsulfoxyde N-{3-[3-cyclopropyl-5-(2-fluoro-4-iodo-phéylamino)-6,8-diméthyl-2,4,7-trioxo-3,4,6,7-tétrahydro-2H-pyrido[4,3-d]pyrimidin-1-yl]phényl}acétamide est premièrement administré à une dose de charge pendant 1 à 3 jours suivi de l'administration d'une dose d'entretien du composé, et/ou le composé évérolimus est premièrement administré à une dose de charge pendant 1 à 3 jours suivi de l'administration d'une dose d'entretien du composé.

**15.** Une combinaison pour une utilisation selon l'une quelconque des revendications 5 à 14, dans laquelle le cancer est soit du type sauvage ou mutant pour Ras/Raf et soit du type sauvage ou mutant pour PI3K/PTEN.

**16.** Une combinaison pour une utilisation selon l'une quelconque des revendication 5 à 14, dans laquelle le cancer est choisi à partir du cerveau (gliomes), glioblastomes, astrocytomes, le glioblastome multiforme, le syndrome de Bannayan-Zonana, la maladie de Cowden, la maladie de Lhermitte-Duclos, du sein, le cancer inflammatoire du sein, la tumeur de Wilm, le sarcome d'Ewing, le rhabdomyosarcome, l'épendymome, le médulloblastome, du côlon, de la tête et du cou, du rein, du poumon, du foie, le melánome, des ovaries, du pancréas, de la prostate, le sarcome, le ostéosarcome, le tumeur osseuse à cellules géantes, de la thyroïde, la leucémie lymphoblastique à cellules T, la leucémie myéloïde chronique, la leucémie lymphoïde chronique, la leucémie à tricholeucocytes, la leucémie lymphoblastique aiguë, la leucémie myéloïde aiguë, la leucémie neutrophilique chronique, la leucémie lymphoblastique aiguë à cellules T, le plasmacytome, la leucémie immunoblastique à grandes cellules, la leucémie des cellules du manteau, le myélome multiple, la leucémie mégacaryoblastique, le myélome multiple, la leucémie mégacaryocytique, la leucémie promyélocytique, l'érythroleucémie, le lymphome malin, le lymphome hodgkinien, le lymphome non-hodgkinien, le lymphome lymphoblastique à cellules T, le lymphome de Burkitt, le lymphome folliculaire, le neuroblastome, le cancer de la vessie, le cancer urothélial, le cancer du poumon, le cancer de la vulve, le cancer du col utérin, le cancer de l'endomètre, le cancer rénal, le mésothéliome, le cancer de l'oesophage, le cancer des glandes salivaires, le cancer hépatocellulaire, le cancer gastrique, le cancer du nasopharynx, le cancer buccal, le cancer de la bouche, la GIST (tumeur stromale gastro-intestinale) et le cancer du testicule.

# Figure 1

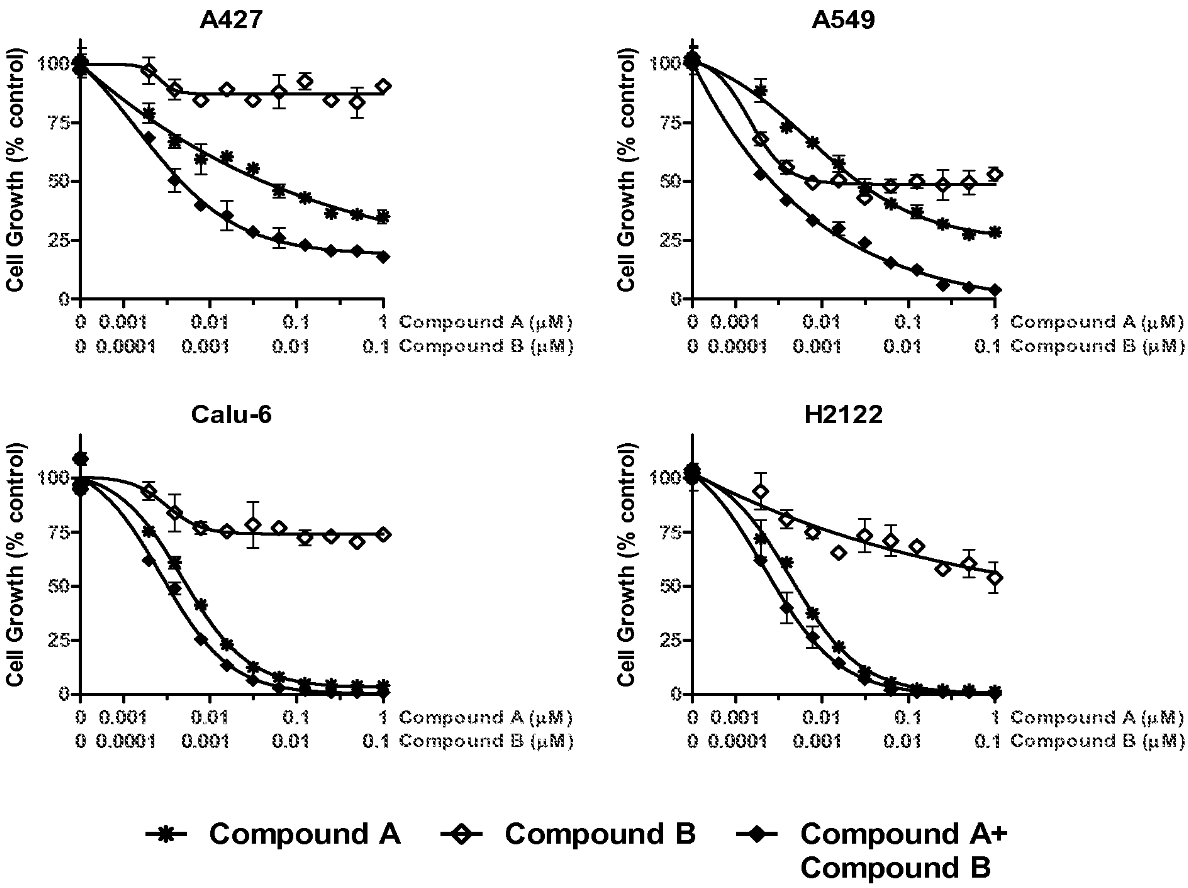
A427
A549
Calu-6
H2122
Cell Growth (% control)
100
75
50
25
0
0 0.001 0.01 0.1 1 Compound A (µM)
0 0.0001 0.001 0.01 0.1 Compound B (µM)
Compound A
Compound B
Compound A+ Compound B

# Figure 2

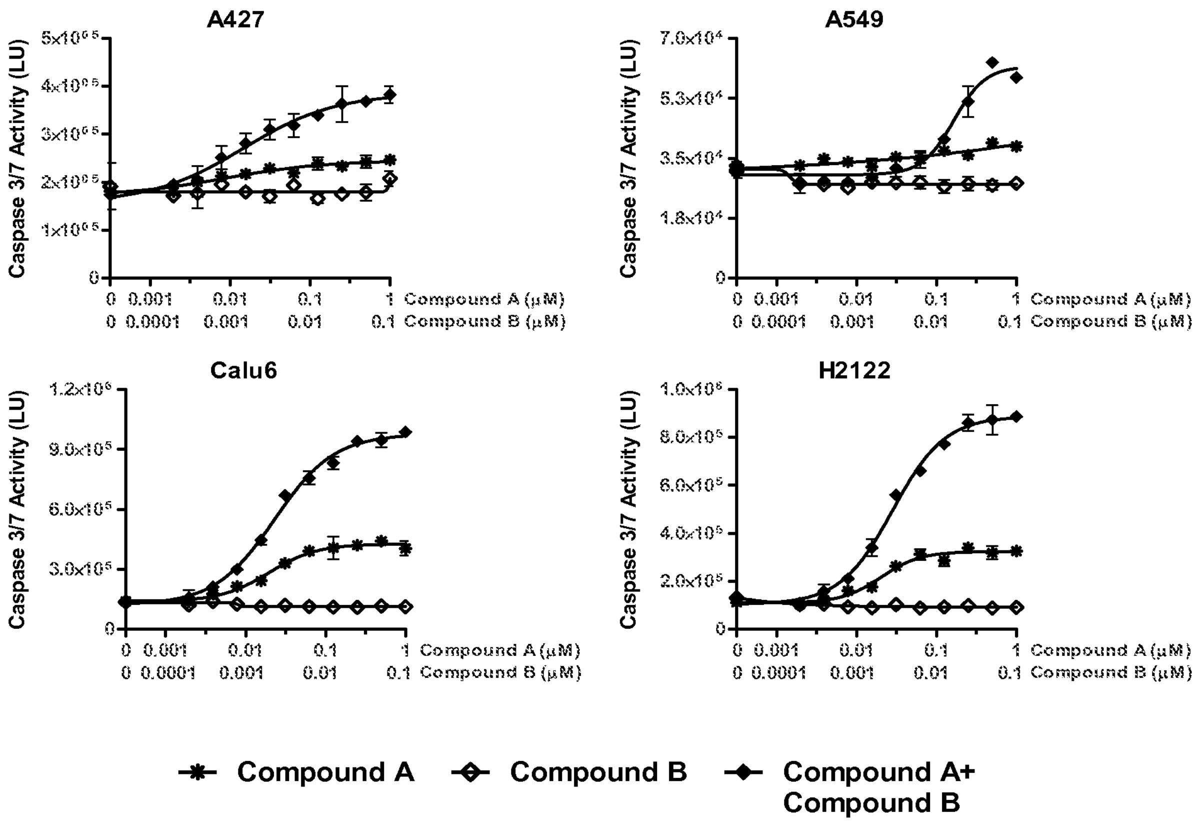
A427
A549
Calu6
H2122
Caspase 3/7 Activity (LU)
Compound A (μM)
Compound B (μM)
0 0.001 0.01 0.1 1
0 0.0001 0.001 0.01 0.1
Compound A
Compound B
Compound A+
Compound B

# Figure 3

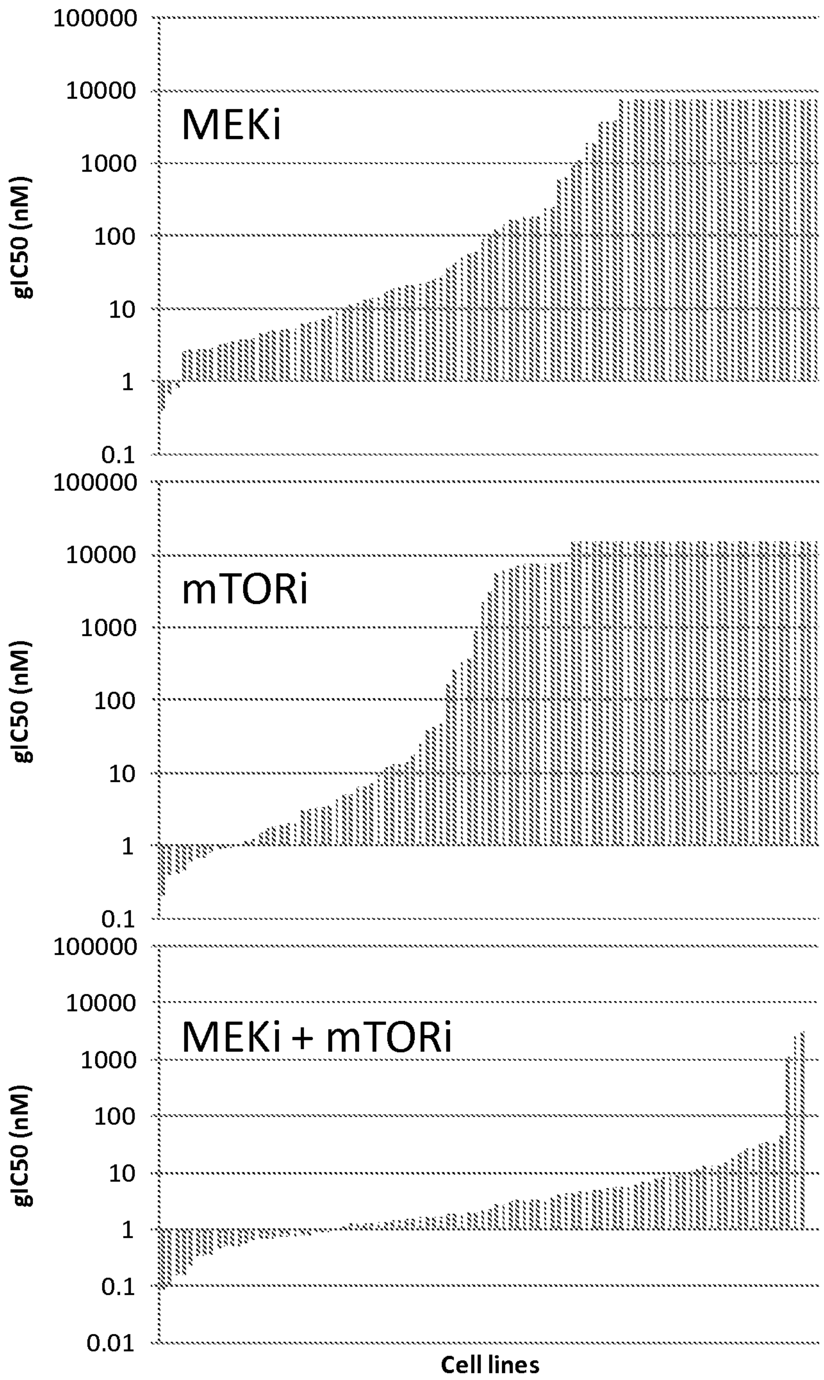
100000
10000
1000
100
10
1
0.1
MEKi
gIC50 (nM)
mTORi
0.01
MEKi + mTORi
Cell lines

# Figure 4

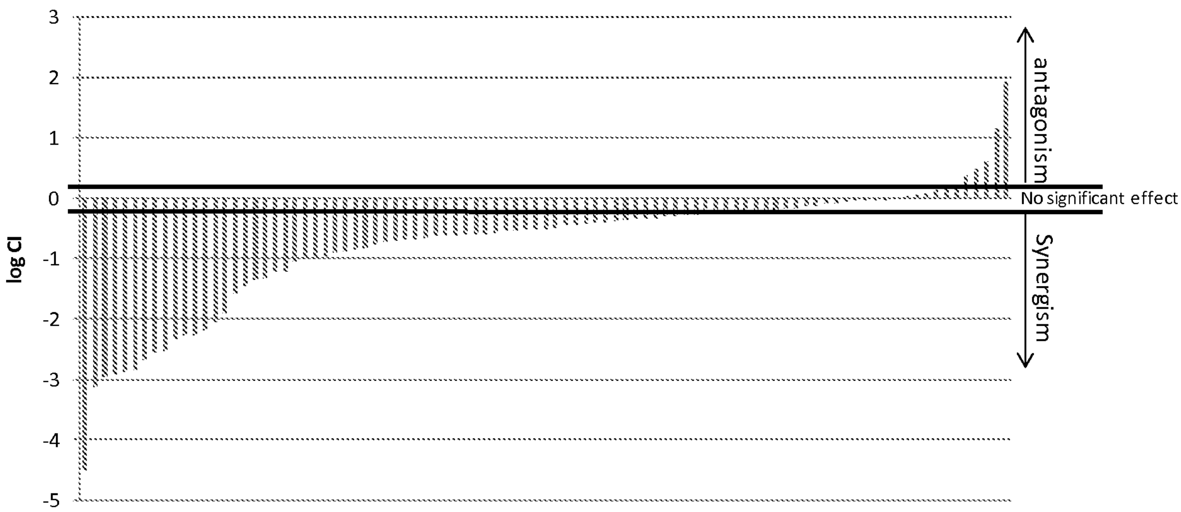
3
2
1
0
-1
-2
-3
-4
-5
log CI
antagonism
No significant effect
Synergism

# Figure 5

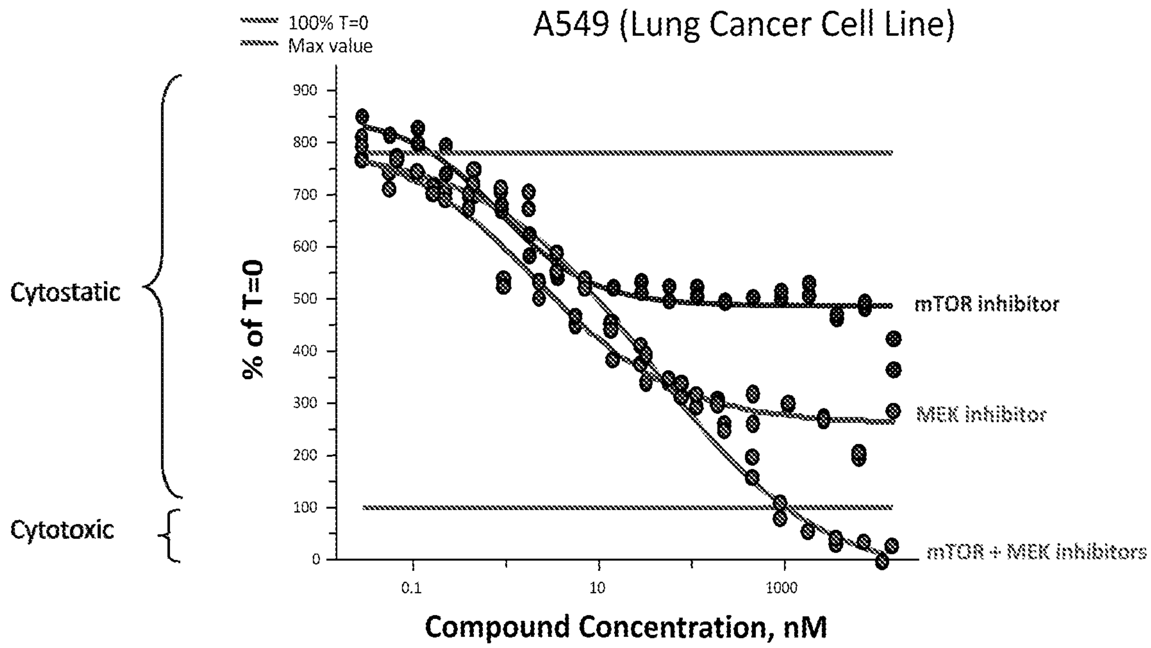
100% T=0
Max value
A549 (Lung Cancer Cell Line)
Cytostatic
Cytotoxic
% of T=0
900
800
700
600
500
400
300
200
100
0
0.1
10
1000
Compound Concentration, nM
mTOR inhibitor
MEK inhibitor
mTOR + MEK inhibitors

# Figure 6

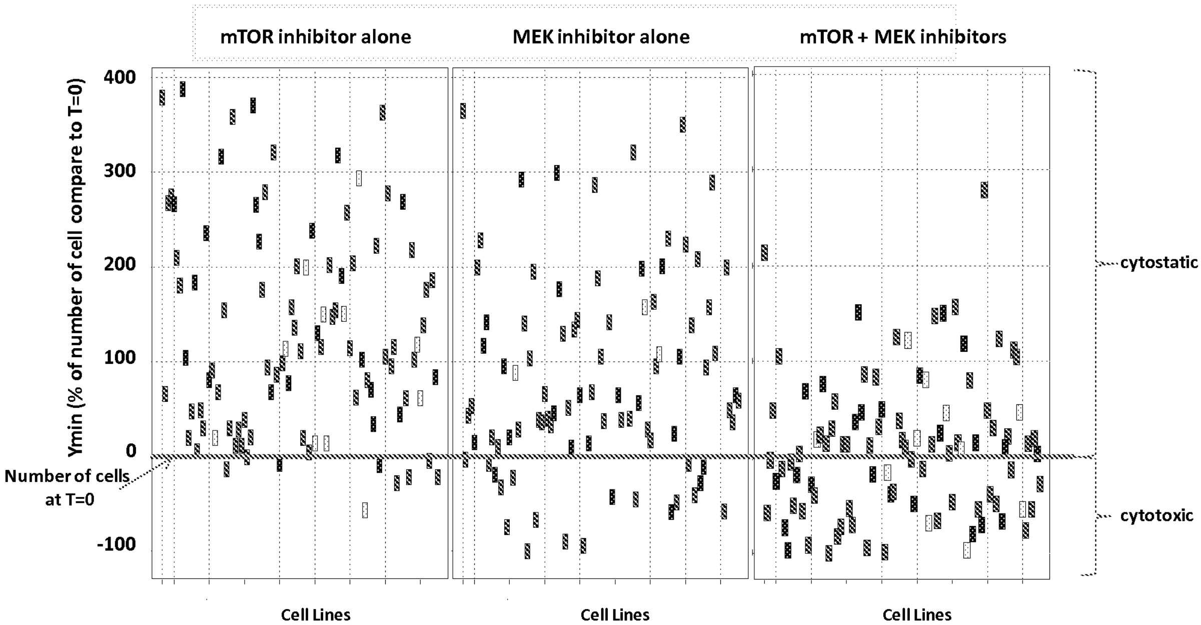


| % of cells with cytotoxic response | | |
|---|---|---|
| mTORi | MEKi | MEK + mTOR i |
| 9.5% | 20% | 44.2% |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2005011082 W
- WO 2005121142 A
- US 20060014768 A
- US 5681835 A
- US 5877219 A
- US 6207716 B
- US 6268391 B

### Non-patent literature cited in the description


- **CREWS ; ERIKSON.** *Cell,* 1993, vol. 74, 215-17
- *The Journal of Biological Chemistry,* 2001, vol. 276 (4), 2686-2692
- **MARKMAN et al.** *Yale Journal of Biology and Medicine,* 1991, vol. 64, 583
- **MCGUIRE et al.** *Ann. Intem, Med.,* 1989, vol. 111, 273
- **HOLMES et al.** *J. Nat. Cancer Inst.,* 1991, vol. 83, 1797
- **EINZIG.** *Proc. Am. Soc. Clin. Oncol.,* vol. 20, 46
- **FORASTIRE.** *Sem. Oncol.,* 1990, vol. 20, 56
- **WOO.** *Nature,* 1994, vol. 368, 750
- **IGNOFF, R.J.** *Cancer Chemotherapy Pocket Guide,* 1998
- **KEARNS, C.M.** *Seminars in Oncology,* 1995, vol. 3 (6), 16-23
- **KATH, JOHN C.** *Exp. Opin. Ther. Patents,* 2000, vol. 10 (6), 803-818
- **SHAWVER et al.** *DDT,* February 1997, vol. 2 (2
- Growth factor receptors as targets. **LOFTS, F. J. et al.** New Molecular Targets for Cancer Chemotherapy. CRC press, 1994
- **SINH, S. ; COREY, S.J.** *Journal of Hematotherapy and Stem Cell Research,* 1999, vol. 8 (5), 465-80
- **BOLEN, J.B. ; BRUGGE, J.S.** *Annual review of Immunology.,* 1997, vol. 15, 371-404
- **SMITHGALL, T.E.** *Journal of Pharmacological and Toxicological Methods,* 1995, vol. 34 (3), 125-32
- **YAMAMOTO, T. ; TAYA, S. ; KAIBUCHI, K.** *Journal of Biochemistry,* 1999, vol. 126 (5), 799-803
- **BRODT, P ; SAMANI, A. ; NAVAB, R.** *Biochemical Pharmacology,* 2000, vol. 60, 1101-1107
- **MASSAGUE, J. ; WEIS-GARCIA, F.** *Cancer Surveys,* 1996, vol. 27, 41-64
- **PHILIP, P.A. ; HARRIS, A.L.** *Cancer Treatment and Research,* 1995, vol. 78, 3-27
- **LACKEY, K. et al.** *Bioorganic and Medicinal Chemistry Letters,* 2000, vol. 10, 223-226
- **MARTINEZ-LACACI, L. et al.** *Int. J. Cancer,* 2000, vol. 88 (1), 44-52
- **ABRAHAM, R.T.** *Current Opinion in Immunology,* 1996, vol. 8 (3), 412-8
- **CANMAN, C.E. ; LIM, D.S.** *Oncogene,* 1998, vol. 17 (25), 3301-3308
- **JACKSON, S.P.** *International Journal of Biochemistry and Cell Biology,* 1997, vol. 29 (7), 935-8
- **ZHONG, H. et al.** *Cancer res,* 2000, vol. 60 (6), 1541-1545
- **POWIS, G. ; KOZIKOWSKI A.** New Molecular Targets for Cancer Chemotherapy. CRC press, 1994
- **SCHAROVSKY, O.G. ; ROZADOS, V.R. ; GERVASONI, S.I. ; MATAR, P.** *Journal of Biomedical Science,* 2000, vol. 7 (4), 292-8
- **ASHBY, M.N.** *Current Opinion in Lipidology,* 1998, vol. 9 (2), 99-102
- *BioChim. Biophys. Acta,* 19 August 1999, vol. 1423 (3), 19-30
- **GREEN, M.C. et al.** *Monoclonal Antibody Therapy for Solid Tumors, Cancer Treat. Rev.,* 2000, vol. 26 (4), 269-286
- *Tyrosine Kinase Signalling in Breast cancer:erbB Family Receptor Tyrosine Kinases, Breast cancer Res.,* 2000, vol. 2 (3), 176-183
- **BREKKEN, R.A. et al.** Selective Inhibition of VEGFR2 Activity by a monoclonal Anti-VEGF antibody blocks tumor growth in mice. *Cancer Res.,* 2000, vol. 60, 5117-5124
- **ROSANIA et al.** *Exp. Opin. Ther. Patents,* 2000, vol. 10 (2), 215-230
- **CHOU ; TALALAY.** *Advances in Enzyme Regulation,* 1984, vol. 22, 37-55
- **BERENBAUM, MC.** *Adv. Cancer Research,* 1981, vol. 35, 269-335
- **BAMFORD S. et al.** *Br. J. Cancer.,* 2004, vol. 91, 355-58

- **PETERSON ; NOVICK SJ.** *J Recept Signal Transduct Res,* 2007, vol. 27, 125-46
- *Peterson J. Frontiers of Bioscience S2,* 2010, 483-503
- **HONGBIN et al.** *Nature,* 2007, vol. 448, 807-810
- **HEARLE et al.** *Clin. Cancer Res.,* 2006, vol. 12, 3209-3215
- *Hum Mutat,* 2005, vol. 26 (4), 291-297
- *Launonen. Human Mutation,* 2005, vol. 26 (4), 291-297